# EUROPÄISCHE PATENTSCHRIFT

(11) **EP 3 548 485 B1**
(45) Veröffentlichungstag und Bekanntmachung des Hinweises auf die Patenterteilung: **20.01.2021**
(21) Anmeldenummer: 17808891.0
(22) Anmeldetag: 04.12.2017
(51) Int. Cl.: C07D 471/04, C07D 471/14, C07D 471/22, C07D 491/04, C07D 495/04, C07F 5/02

(54) **MATERIALIEN FÜR ORGANISCHE ELEKTROLUMINESZENZVORRICHTUNGEN**
MATERIALS FOR ORGANIC ELECTROLUMINESCENT DEVICES
MATÉRIAUX POUR DISPOSITIFS ÉLECTROLUMINESCENTS ORGANIQUES

(30) Priorität: 05.12.2016 EP 16202121
(43) Veröffentlichungstag der Anmeldung: 09.10.2019
(73) Patentinhaber: Merck Patent GmbH, 64293 Darmstadt (DE)
(72) Erfinder: STOESSEL, Philipp, 60389 Frankfurt am Main (DE); PARHAM, Amir, 60486 Frankfurt am Main (DE); JOOSTEN, Dominik, 60487 Frankfurt am Main (DE); LUDEMANN, Aurélie, 60322 Frankfurt am Main (DE); GROSSMANN, Tobias, 64297 Darmstadt (DE); KROEBER, Jonas, 60311 Frankfurt am Main (DE)
(86) Internationale Anmeldenummer: PCT/EP2017/081288
(87) Internationale Veröffentlichungsnummer: WO 2018/104193

(56) Entgegenhaltungen:
- US-A1- 2013 112 946
- US-A1- 2016 190 449
- DATABASE CA [Online] CHEMICAL ABSTRACTS SERVICE, COLUMBUS, OHIO, US; MOSZEW, JAN ET AL: "Heterocyclic analogues of carcinogenic hydrocarbons. Polymethyl derivatives of diazabenzanthracene. Triazaphenalene and quinoline. II", XP002777268, gefunden im STN Database accession no. 1971:53592
- DATABASE CA [Online] CHEMICAL ABSTRACTS SERVICE, COLUMBUS, OHIO, US; MOSZEW, JAN ET AL: "Heterocyclic analogs of carcinogenic hydrocarbons. Polymethyl derivatives of diazabenzanthracene and of triazaphenalene. I", XP002777269, gefunden im STN Database accession no. 1970:43519
- DATABASE CA [Online] CHEMICAL ABSTRACTS SERVICE, COLUMBUS, OHIO, US; MOSZEW, JAN ET AL: "Structure and properties of triazaphenalene, a heterocyclic system of condensed rings", XP002777270, gefunden im STN Database accession no. 1968:486850
- DATABASE CA [Online] CHEMICAL ABSTRACTS SERVICE, COLUMBUS, OHIO, US; MOSZEW, JAN ET AL: "Heterocyclic analogs of carcinogenic hydrocarbons. New derivatives of diazabenzanthracene and triazaphenalene", XP002777271, gefunden im STN Database accession no. 1968:410388
- DATABASE CA [Online] CHEMICAL ABSTRACTS SERVICE, COLUMBUS, OHIO, US; MOSZEW, JAN ET AL: "Absorption of visible and ultraviolet light by some derivatives of diazabenzanthrone and triazaphenalene", XP002777272, gefunden im STN Database accession no. 1968:410030
- DATABASE CA [Online] CHEMICAL ABSTRACTS SERVICE, COLUMBUS, OHIO, US; MOSZEW, J. ET AL: "Heterocyclic analogs of carcinogenic hydrocarbons as potential anticarcinogens", XP002777273, gefunden im STN Database accession no. 1965:502356
- DATABASE CA [Online] CHEMICAL ABSTRACTS SERVICE, COLUMBUS, OHIO, US; MOSZEW, J. ET AL: "Absorption properties of some heterocyclic analogs of carcinogenic hydrocarbons in the visible and ultraviolet range", XP002777274, gefunden im STN Database accession no. 1965:488232
- DATABASE CA [Online] CHEMICAL ABSTRACTS SERVICE, COLUMBUS, OHIO, US; MOSZEW, J. ET AL: "Characteristic isomerism and transformations of derivatives of 1,2-benzo-3,9-diazaanthracene", XP002777275, gefunden im STN Database accession no. 1965:43862

## Beschreibung

Die vorliegende Erfindung betrifft Materialien für die Verwendung in elektronischen Vorrichtungen, insbesondere in organischen Elektrolumineszenzvorrichtungen, sowie elektronische Vorrichtungen, insbesondere organische Elektrolumineszenzvorrichtungen enthaltend diese Materialien.

In organischen Elektrolumineszenzvorrichtungen (OLEDs) werden als emittierende Materialien häufig metallorganische Komplexe eingesetzt, die Phosphoreszenz statt Fluoreszenz zeigen. Aus quantenmechanischen Gründen ist unter Verwendung metallorganischer Verbindungen als Phosphoreszenzemitter eine bis zu vierfache Energie- und Leistungseffizienz möglich. Generell gibt es bei OLEDs, insbesondere auch bei OLEDs, die Triplettemission (Phosphoreszenz) zeigen, jedoch immer noch Verbesserungsbedarf, beispielsweise im Hinblick auf Effizienz, Betriebsspannung und Lebensdauer. Die Eigenschaften phosphoreszierender OLEDs werden nicht nur von den eingesetzten Triplettemittern bestimmt. Hier sind insbesondere auch die anderen verwendeten Materialien, wie Matrixmaterialien, von besonderer Bedeutung. Verbesserungen dieser Materialien und deren Ladungstransporteigenschaften können somit auch zu deutlichen Verbesserungen der OLED-Eigenschaften führen.

Aufgabe der vorliegenden Erfindung ist die Bereitstellung von Verbindungen, welche sich für den Einsatz in einer OLED eignen, insbesondere als Matrixmaterial für phosphoreszierende Emitter. Eine weitere Aufgabe der vorliegenden Erfindung ist es, weitere organische Halbleiter für organische Elektrolumineszenzvorrichtungen bereitzustellen, um so dem Fachmann eine größere Wahlmöglichkeit an Materialien für die Herstellung von OLEDs zu ermöglichen.

Überraschend wurde gefunden, dass bestimmte, unten näher beschriebene Verbindungen diese Aufgabe lösen, sich gut für die Verwendung in OLEDs eignen und zu Verbesserungen der organischen Elektrolumineszenzvorrichtung führen. Dabei betreffen die Verbesserungen insbesondere die Lebensdauer, die Effizienz und/oder die Betriebsspannung.

Diese Verbindungen sowie elektronische Vorrichtungen, insbesondere organische Elektrolumineszenzvorrichtungen, welche derartige Verbindungen enthalten, sind daher der Gegenstand der vorliegenden Erfindung.

Aus US 2016/0190449 sind pentacyclische aromatische Verbindungen für OLEDs bekannt, die mit mindestens einem Diarylboran-Substituenten substituiert sind. Aus US 2013/0112946 sind hexacyclische aromatische Verbindungen für die Verwendung in OLEDs bekannt. Entsprechende pentacyclische Verbindungen sind nicht offenbart.

Gegenstand der vorliegenden Erfindung ist daher eine Verbindung gemäß Formel (1), 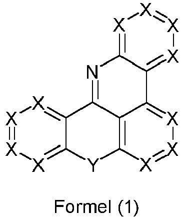 wobei für die verwendeten Symbole gilt:
- X: ist bei jedem Auftreten gleich oder verschieden CR oder N;
- Y: ist NR', BR', O oder S;
- R: ist bei jedem Auftreten gleich oder verschieden H, D, F, Cl, Br, I, NAr₂, N(R¹)₂, CN, NO₂, OR¹, SR¹, COOR¹, C(=O)N(R¹)₂, Si(R¹)₃, B(OR¹)₂, C(=O)R¹, P(=O)(R¹)₂, S(=O)R¹, S(=O)₂R¹, OSO₂R1, eine geradkettige Alkylgruppe mit 1 bis 20 C-Atomen oder eine Alkenyl- oder Alkinylgruppe mit 2 bis 20 C-Atomen oder eine verzweigte oder cyclische Alkylgruppe mit 3 bis 20 C-Atomen, wobei die Alkyl-, Alkenyl- oder Alkinylgruppe jeweils mit einem oder mehreren Resten R¹ substituiert sein kann und wobei eine oder mehrere nicht benachbarte CH₂-Gruppen durch Si(R¹)₂, C=O, NR¹, O, S oder CONR¹ ersetzt sein können, oder ein aromatisches oder heteroaromatisches Ringsystem mit 5 bis 60 aromatischen Ringatomen, bevorzugt mit 5 bis 40 aromatischen Ringatomen, das jeweils durch einen oder mehrere Reste R¹ substituiert sein kann; dabei können zwei Reste R auch miteinander ein aliphatisches oder heteroaliphatisches Ringsystem bilden;
- R': ist ein aromatisches oder heteroaromatisches Ringsystem mit 5 bis 40 aromatischen Ringatomen, das durch einen oder mehrere Reste R¹ substituiert sein kann;
- Ar: ist bei jedem Auftreten gleich oder verschieden ein aromatisches oder heteroaromatisches Ringsystem mit 5 bis 40 aromatischen Ringatomen, das durch einen oder mehrere Reste R¹ substituiert sein kann;
- R¹: ist bei jedem Auftreten gleich oder verschieden H, D, F, Cl, Br, I, N(R²)₂, CN, NO₂, OR², SR², Si(R²)₃, B(OR²)₂, C(=O)R², P(=O)(R²)₂, S(=O)R², S(=O)₂R², OSO₂R², eine geradkettige Alkylgruppe mit 1 bis 20 C-Atomen oder eine Alkenyl- oder Alkinylgruppe mit 2 bis 20 C-Atomen oder eine verzweigte oder cyclische Alkylgruppe mit 3 bis 20 C-Atomen, wobei die Alkyl-, Alkenyl- oder Alkinylgruppe jeweils mit einem oder mehreren Resten R² substituiert sein kann und wobei eine oder mehrere nicht benachbarte CH₂-Gruppen durch Si(R²)₂, C=O, NR², O, S oder CONR² ersetzt sein können, oder ein aromatisches oder heteroaromatisches Ringsystem mit 5 bis 40 aromatischen Ringatomen, das jeweils durch einen oder mehrere Reste R² substituiert sein kann; dabei können zwei oder mehrere Reste R¹ miteinander ein Ringsystem bilden;
- R²: ist bei jedem Auftreten gleich oder verschieden H, D, F oder ein aliphatischer, aromatischer oder heteroaromatischer organischer Rest, insbesondere ein Kohlenwasserstoffrest, mit 1 bis 20 C-Atomen, in dem auch ein oder mehrere H-Atome durch F ersetzt sein können;
mit der Maßgabe, dass die Verbindung der Formel (1) mindestens einen Substituenten R' enthält und/oder dass die Verbindung der Formel (1) mindestens einen Substituenten R enthält, der ausgewählt ist aus der Gruppe bestehend aus NAr₂ oder einem aromatischen oder heteroaromatischen Ringsystem mit 5 bis 60 aromatischen Ringatomen, welches durch einen oder mehrere Reste R¹ substituiert sein kann.

Eine Arylgruppe im Sinne dieser Erfindung enthält 6 bis 40 C-Atome; eine Heteroarylgruppe im Sinne dieser Erfindung enthält 2 bis 40 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte (anellierte) Aryl- oder Heteroarylgruppe, beispielsweise Naphthalin, Anthracen, Phenanthren, Chinolin, Isochinolin, etc., verstanden. Miteinander durch Einfachbindung verknüpfte Aromaten, wie zum Beispiel Biphenyl, werden dagegen nicht als Aryl- oder Heteroarylgruppe, sondern als aromatisches Ringsystem bezeichnet.

Ein aromatisches Ringsystem im Sinne dieser Erfindung enthält 6 bis 60 C-Atome im Ringsystem. Ein heteroaromatisches Ringsystem im Sinne dieser Erfindung enthält 2 bis 60 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit, wie z. B. ein C-, N- oder O-Atom, verbunden sein können. So sollen beispielsweise auch Systeme wie Fluoren, 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine kurze Alkylgruppe verbunden sind.

Im Rahmen der vorliegenden Erfindung werden unter einem aliphatischen Kohlenwasserstoffrest bzw. einer Alkylgruppe bzw. einer Alkenyl- oder Alkinylgruppe, die 1 bis 40 C-Atome enthalten kann, und in der auch einzelne H-Atome oder CH₂-Gruppen durch die oben genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, neo-Pentyl, Cyclopentyl, n-Hexyl, neo-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl, Heptinyl oder Octinyl verstanden. Unter einer Alkoxygruppe OR¹ mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy und 2,2,2-Trifluorethoxy verstanden. Unter einer Thioalkylgruppe SR¹ mit 1 bis 40 C-Atomen werden insbesondere Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden. Allgemein können Alkyl-, Alkoxy- oder Thioalkylgruppen gemäß der vorliegenden Erfindung geradkettig, verzweigt oder cyclisch sein, wobei eine oder mehrere nicht-benachbarte CH₂-Gruppen durch die oben genannten Gruppen ersetzt sein können; weiterhin können auch ein oder mehrere H-Atome durch D, F, Cl, Br, I, CN oder NO₂, bevorzugt F, Cl oder CN, weiter bevorzugt F oder CN, besonders bevorzugt CN ersetzt sein.

Unter einem aromatischen oder heteroaromatischen Ringsystem mit 5 - 60 aromatischen Ringatomen, welches noch jeweils mit den oben genannten Resten R² oder einem Kohlenwasserstoffrest substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Pyren, Chrysen, Perylen, Fluoranthen, Naphthacen, Pentacen, Benzpyren, Biphenyl, Biphenylen, Terphenyl, Triphenylen, Fluoren, Spirobifluoren, Dihydrophenanthren, Dihydropyren, Tetrahydropyren, cis- oder trans-Indenofluoren, cis- oder trans-Indenocarbazol, cis- oder trans-Indolocarbazol, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Hexaazatriphenylen, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Gruppen, die abgeleitet sind von Kombination dieser Systeme.

Wenn zwei Reste R bzw. R¹ miteinander ein Ringsystem bilden, so kann dieses mono- oder polycyclisch, aliphatisch, heteroaliphatisch bzw. für Reste R¹ auch aromatisch oder heteroaromatisch sein. Dabei sind die Reste, die miteinander ein Ringsystem bilden, bevorzugt benachbart, d. h. dass diese Reste an dasselbe Kohlenstoffatom oder an Kohlenstoffatome, die direkt aneinander gebunden sind, gebunden sind.

Unter der Formulierung, dass zwei oder mehr Reste miteinander einen Ring bilden können, soll im Rahmen der vorliegenden Beschreibung unter anderem verstanden werden, dass die beiden Reste miteinander durch eine chemische Bindung unter formaler Abspaltung von zwei Wasserstoffatomen verknüpft sind. Dies wird durch das folgende Schema verdeutlicht.

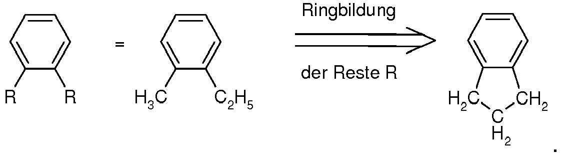

Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet. Dies soll durch das folgende Schema verdeutlicht werden:

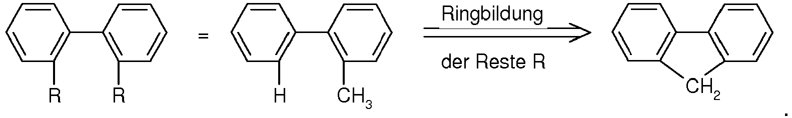

In einer bevorzugten Ausführungsform der Erfindung steht Y für NR', O oder S, besonders bevorzugt für NR' oder O und ganz besonders bevorzugt für NR'.

Bevorzugt handelt es sich somit um eine Verbindung gemäß einer der folgenden Formeln (1') oder (1"), 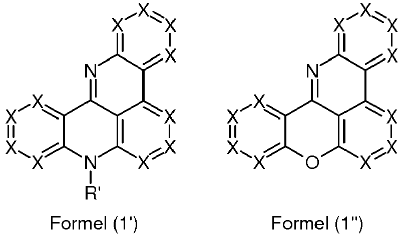 wobei X die oben genannten Bedeutungen aufweist und die Verbindung der Formel (1 ") mindestens einen Rest R aufweist, der ausgewählt ist aus der Gruppe bestehend aus NAr₂ und einem aromatischen oder heteroaromatischen Ringsystem. Dabei ist die Verbindung der Formel (1') besonders bevorzugt.

In einer bevorzugten Ausführungsform der Verbindungen der Formel (1), (1') oder (1") stehen maximal zwei Symbole X, bevorzugt maximal ein Symbol X, pro Ring für N, und die verbleibenden Symbole X stehen für CR. Besonders bevorzugt stehen insgesamt maximal zwei Symbole X, insbesondere maximal ein Symbol X für N. Ganz besonders bevorzugt stehen alle Symbole X für CR.

Bevorzugt sind die Verbindungen der Formel (2a) bis (2h), 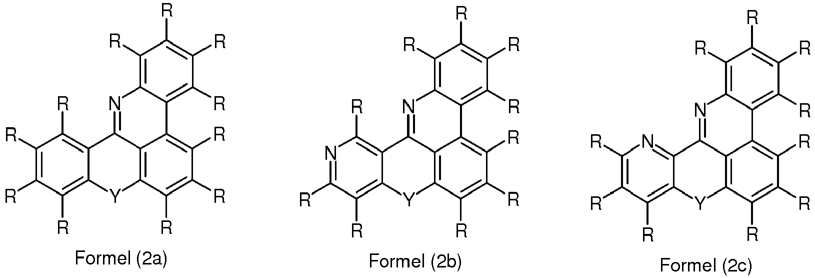 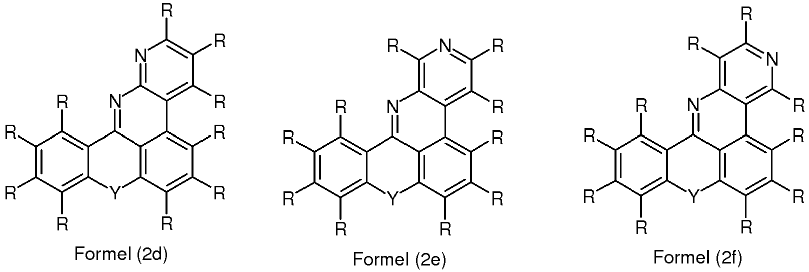 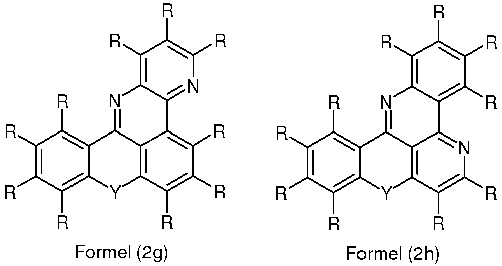 wobei die verwendeten Symbole die oben genannten Bedeutungen aufweisen. Dabei sind die Verbindungen der Formeln (2a) bis (2f) und (2h) bevorzugt für Y = O, und die Verbindungen der Formeln (2a), (2b) und (2d) bis (2h) sind bevorzugt für Y = NR'. Besonders bevorzugt sind die Verbindungen der Formel (2a).

Besonders bevorzugt sind die Verbindungen der Formeln (2a') und (2a"), 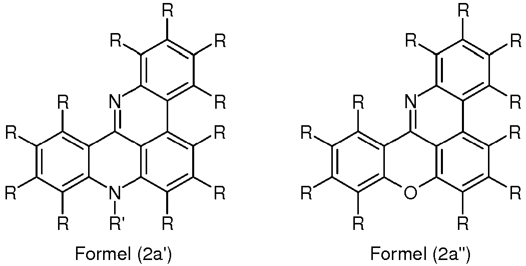 wobei R und R' die oben genannten Bedeutungen aufweisen und mindestens eine Gruppe R in Formel (2a") ausgewählt ist aus der Gruppe bestehend aus NAr₂ und einem aromatischen oder heteroaromatischen Ringsystem.

Eine bevorzugte Ausführungsform der Verbindungen der Formel (2a) sind die Verbindungen der folgenden Formel (3), 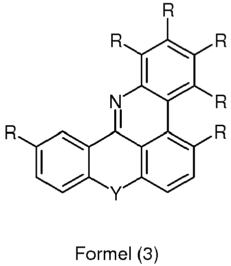 wobei die verwendeten Symbole die oben genannten Bedeutungen aufweisen.

Besonders bevorzugt sind die Verbindungen der Formeln (3') und (3"), 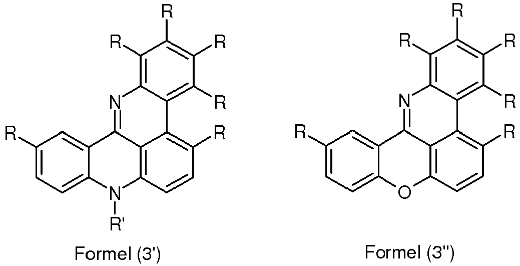 wobei R und R' die oben genannten Bedeutungen aufweisen und mindestens eine Gruppe R in Formel (3") ausgewählt ist aus der Gruppe bestehend aus NAr₂ und einem aromatischen oder heteroaromatischen Ringsystem.

Eine bevorzugte Ausführungsform der Verbindungen der Formel (3) sind die Verbindungen der folgenden Formel (4), 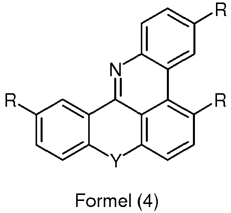 wobei die verwendeten Symbole die oben genannten Bedeutungen aufweisen.

Besonders bevorzugt sind die Verbindungen der Formeln (4') und (4"), 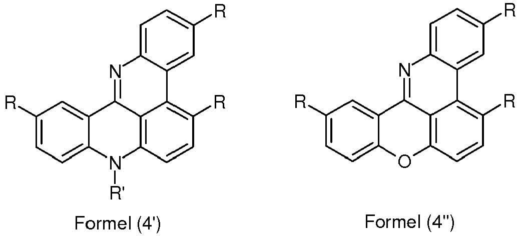 wobei R und R' die oben genannten Bedeutungen aufweisen und mindestens eine Gruppe R in Formel (4") ausgewählt ist aus der Gruppe bestehend aus NAr₂ und einem aromatischen oder heteroaromatischen Ringsystem.

Im Folgenden werden bevorzugte Substituenten R und R' an den erfindungsgemäßen Verbindungen beschrieben.

In einer bevorzugten Ausführungsform der Erfindung ist R bei jedem Auftreten gleich oder verschieden ausgewählt aus der Gruppe bestehend aus H, D, F, NAr₂, CN, OR¹, einer geradkettigen Alkylgruppe mit 1 bis 10 C-Atomen oder einer Alkenylgruppe mit 2 bis 10 C-Atomen oder einer verzweigten oder cyclischen Alkylgruppe mit 3 bis 10 C-Atomen, wobei die Alkyl- bzw. Alkenylgruppe jeweils mit einem oder mehreren Resten R¹ substituiert sein kann und wobei eine oder mehrere nicht benachbarte CH₂-Gruppen durch O ersetzt sein können, oder einem aromatischen oder heteroaromatischen Ringsystem mit 6 bis 30 aromatischen Ringatomen, das jeweils durch einen oder mehrere Reste R¹ substituiert sein kann; dabei können zwei Reste R auch miteinander ein aliphatisches Ringsystem bilden. Besonders bevorzugt ist R bei jedem Auftreten gleich oder verschieden ausgewählt aus der Gruppe bestehend aus H, NAr₂, einer geradkettigen Alkylgruppe mit 1 bis 6 C-Atomen, insbesondere mit 1, 2, 3 oder 4 C-Atomen, oder einer verzweigten oder cyclischen Alkylgruppe mit 3 bis 6 C-Atomen, wobei die Alkylgruppe jeweils mit einem oder mehreren Resten R¹ substituiert sein kann, bevorzugt aber unsubstituiert ist, oder einem aromatischen oder heteroaromatischen Ringsystem mit 6 bis 24 aromatischen Ringatomen, das jeweils durch einen oder mehrere, bevorzugt nicht-aromatische, Reste R¹ substituiert sein kann. Ganz besonders bevorzugt ist R bei jedem Auftreten gleich oder verschieden ausgewählt aus der Gruppe bestehend aus H, NAr₂ oder einem aromatischen oder heteroaromatischen Ringsystem mit 6 bis 24 aromatischen Ringatomen, insbesondere mit 6 bis 13 aromatischen Ringatomen, das jeweils durch einen oder mehrere, bevorzugt nicht-aromatische, Reste R¹ substituiert sein kann.

In einer weiteren bevorzugten Ausführungsform der Erfindung ist R' ein aromatisches oder heteroaromatisches Ringsystem mit 6 bis 30 aromatischen Ringatomen, das durch einen oder mehrere Reste R¹ substituiert sein kann. In einer besonders bevorzugten Ausführungsform der Erfindung ist R' ein aromatisches oder heteroaromatisches Ringsystem mit 6 bis 24 aromatischen Ringatomen, insbesondere mit 6 bis 13 aromatischen Ringatomen, das durch einen oder mehrere, bevorzugt nicht-aromatische, Reste R¹ substituiert sein kann.

In einer weiteren bevorzugten Ausführungsform der Erfindung ist R¹ gleich oder verschieden bei jedem Auftreten ausgewählt aus der Gruppe bestehend aus H, D, F, CN, OR², einer geradkettigen Alkylgruppe mit 1 bis 10 C-Atomen oder einer Alkenylgruppe mit 2 bis 10 C-Atomen oder einer verzweigten oder cyclischen Alkylgruppe mit 3 bis 10 C-Atomen, wobei die Alkyl- bzw. Alkenylgruppe jeweils mit einem oder mehreren Resten R² substituiert sein kann und wobei eine oder mehrere nicht benachbarte CH₂-Gruppen durch O ersetzt sein können, oder einem aromatischen oder heteroaromatischen Ringsystem mit 6 bis 30 aromatischen Ringatomen, das jeweils durch einen oder mehrere Reste R² substituiert sein kann; dabei können zwei oder mehrere Reste R¹ miteinander ein aliphatisches Ringsystem bilden. In einer besonders bevorzugten Ausführungsform der Erfindung ist R¹ gleich oder verschieden bei jedem Auftreten ausgewählt aus der Gruppe bestehend aus H, einer geradkettigen Alkylgruppe mit 1 bis 6 C-Atomen, insbesondere mit 1, 2, 3 oder 4 C-Atomen, oder einer verzweigten oder cyclischen Alkylgruppe mit 3 bis 6 C-Atomen, wobei die Alkylgruppe mit einem oder mehreren Resten R² substituiert sein kann, bevorzugt aber unsubstituiert ist, oder einem aromatischen oder heteroaromatischen Ringsystem mit 6 bis 24 aromatischen Ringatomen, insbesondere mit 6 bis 13 aromatischen Ringatomen, das jeweils durch einen oder mehrere Reste R² substituiert sein kann, bevorzugt aber unsubstituiert ist.

In einer weiteren bevorzugten Ausführungsform der Erfindung ist R² gleich oder verschieden bei jedem Auftreten H, eine Alkylgruppe mit 1 bis 4 C-Atomen oder eine Arylgruppe mit 6 bis 10 C-Atomen, welche mit einer Alkylgruppe mit 1 bis 4 C-Atomen substituiert sein kann, bevorzugt aber unsubstituiert ist.

Wie oben beschrieben, enthält die erfindungsgemäße Verbindung mindestens eine Gruppe R' und/oder enthält mindestens eine Gruppe R, welche ausgewählt ist aus der Gruppe bestehend aus NAr₂ und einem aromatischen oder heteroaromatischen Ringsystem mit 5 bis 60 aromatischen Ringatomen, welches durch einen oder mehrere Reste R¹ substituiert sein kann. In einer Ausführungsform der Erfindung ist Y = NR' und das Grundgerüst ist unsubstituiert, das heißt R = H. In einer weiteren Ausführungsform der Erfindung ist Y = NR' und das Grundgerüst ist mit mindestens einem Substituenten substituiert, welcher ausgewählt ist aus der Gruppe bestehend aus NAr₂ und einem aromatischen oder heteroaromatischen Ringsystem mit 5 bis 60 aromatischen Ringatomen, welches durch einen oder mehrere Reste R¹ substituiert sein kann. Nachfolgend werden bevorzugte aromatische und heteroaromatische Ringsysteme beschrieben, welche als Substituent R und/oder R' oder als Gruppe Ar innerhalb des Substituenten NAr₂ in der erfindungsgemäßen Verbindung vorliegen können.

Geeignete aromatische bzw. heteroaromatische Ringsystem R, R' bzw. Ar sind ausgewählt aus Phenyl, Biphenyl, insbesondere ortho-, meta- oder para-Biphenyl, Terphenyl, insbesondere ortho-, meta-, para- oder verzweigtem Terphenyl, Quaterphenyl, insbesondere ortho-, meta-, para- oder verzweigtem Quaterphenyl, Fluoren, welches über die 1-, 2-, 3- oder 4-Position verknüpft sein kann, Spirobifluoren, welches über die 1-, 2-, 3- oder 4-Position verknüpft sein kann, Naphthalin, insbesondere 1- oder 2-verknüpftem Naphthalin, Indol, Benzofuran, Benzothiophen, Carbazol, welches über die 1-, 2-, 3- oder 4-Position verknüpft sein kann, Dibenzofuran, welches über die 1-, 2-, 3- oder 4-Position verknüpft sein kann, Dibenzothiophen, welches über die 1-, 2-, 3- oder 4-Position verknüpft sein kann, Indenocarbazol, Indolocarbazol, Pyridin, Pyrimidin, Pyrazin, Pyridazin, Triazin, Phenanthren, Triphenylen oder einer Kombination aus zwei oder drei dieser Gruppen, welche jeweils mit einem oder mehreren Resten R¹ substituiert sein können.

Dabei sind die Gruppen R, R' bzw. Ar bevorzugt gewählt aus den Gruppen der folgenden Formeln Ar-1 bis Ar-75, 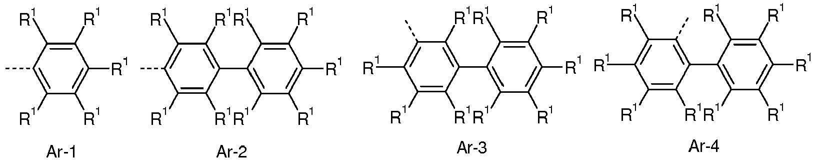 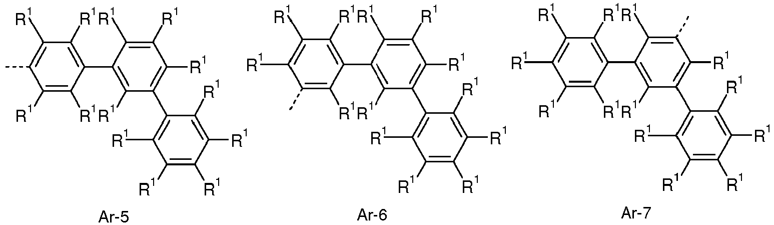 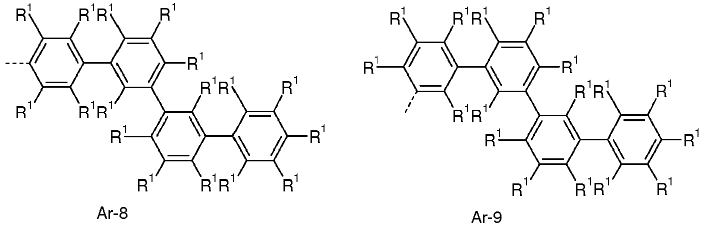 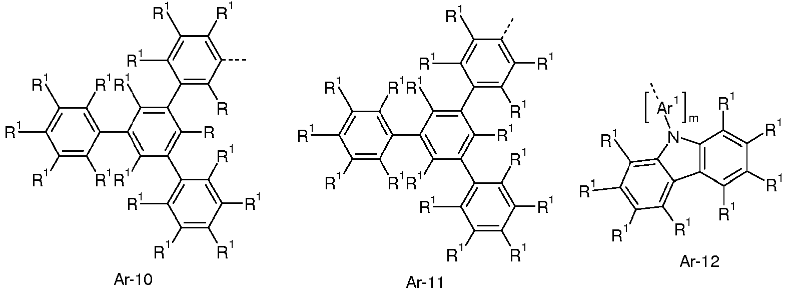 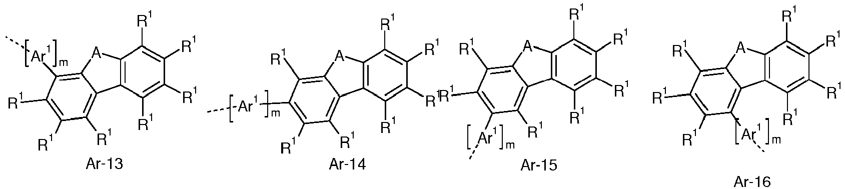 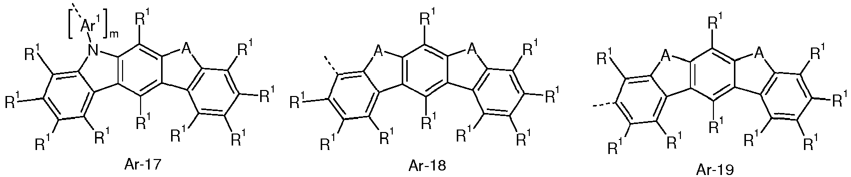 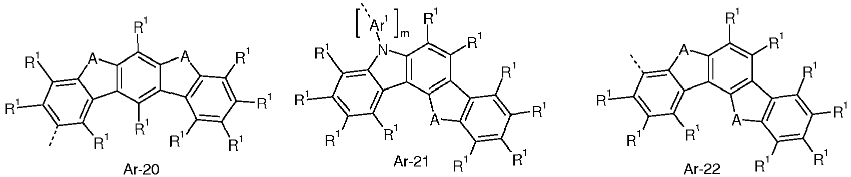 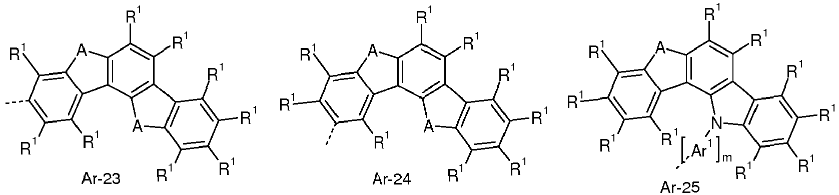 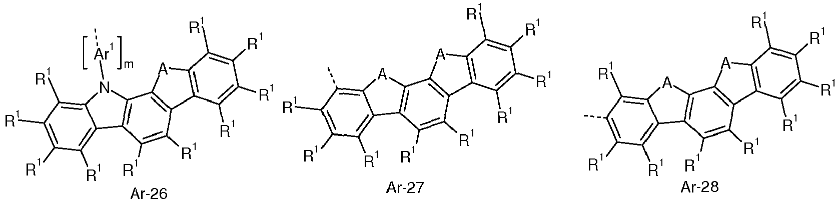 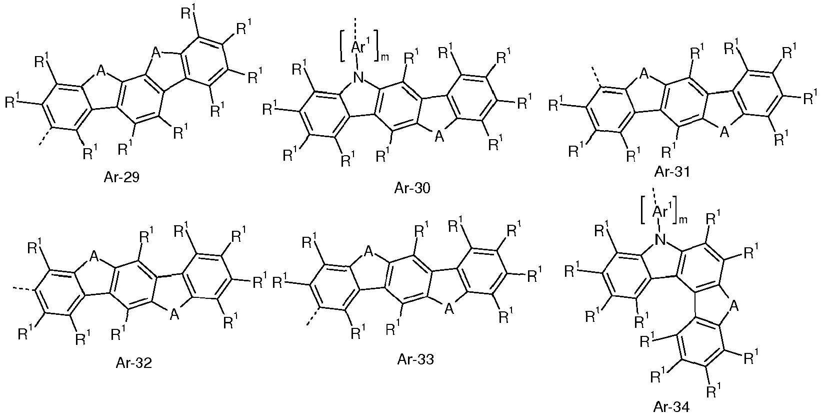 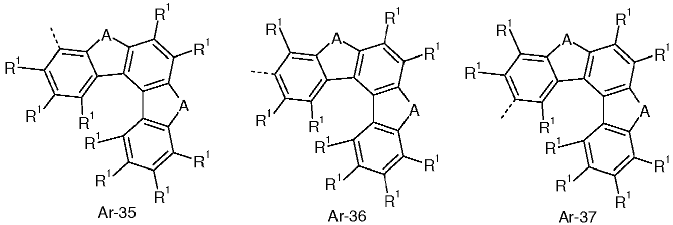 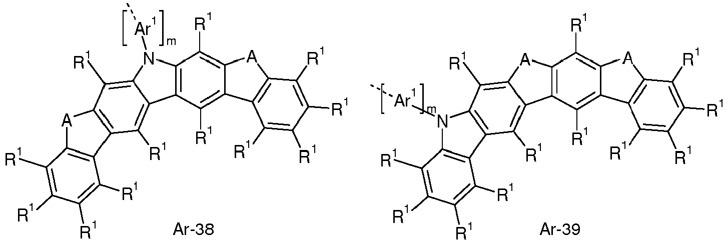 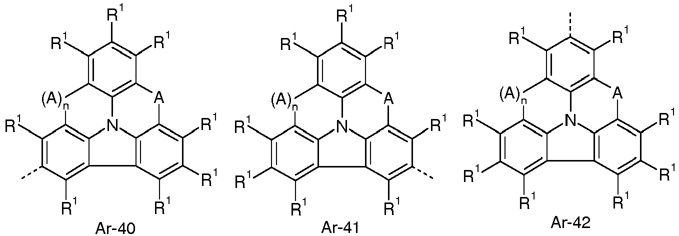 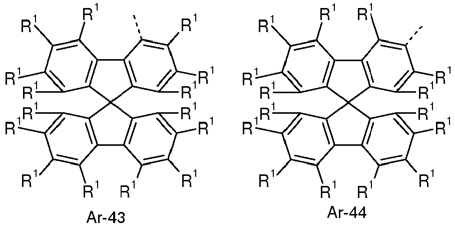 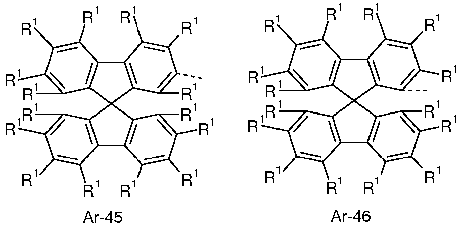 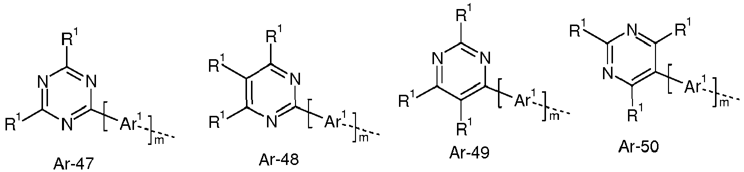 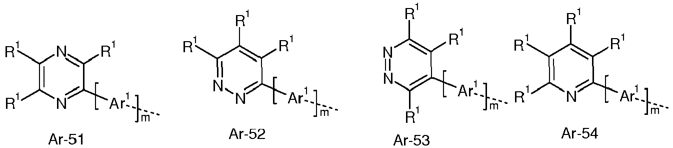 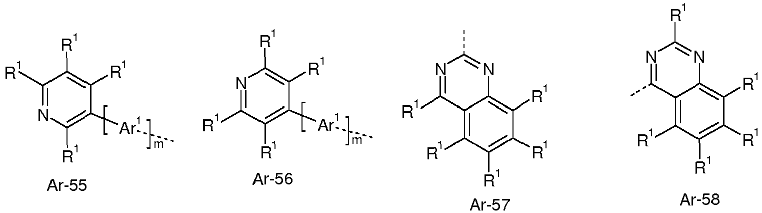 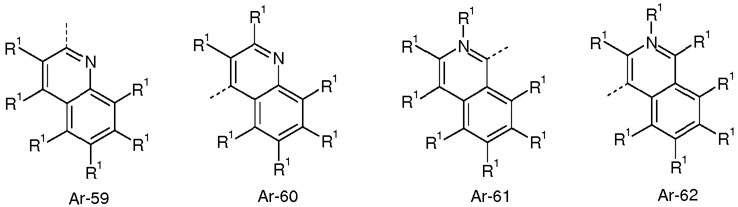 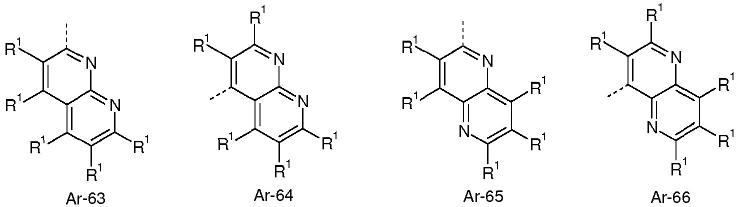 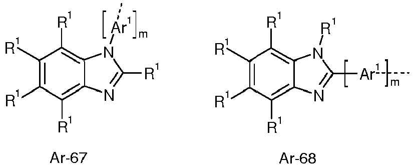 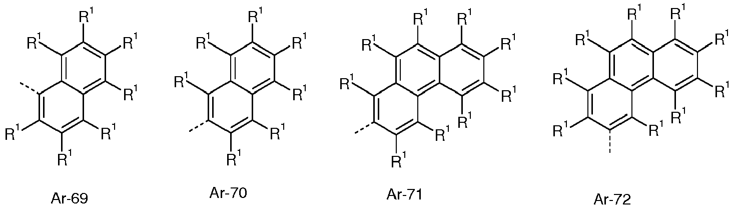 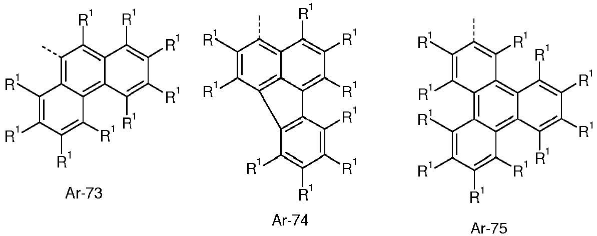 wobei R¹ die oben genannten Bedeutungen aufweist, die gestrichelte Bindung die Bindung an Y bzw. an ein Kohlenstoffatom des Grundgerüsts in Formel (1) bzw. in den bevorzugten Ausführungsformen bzw. an das Stickstoffatom in der Gruppe NAr₂ darstellt und weiterhin gilt:
- Ar¹: ist bei jedem Auftreten gleich oder verschieden ein bivalentes aromatisches oder heteroaromatisches Ringsystem mit 6 bis 18 aromatischen Ringatomen, welches jeweils mit einem oder mehreren Resten R¹ substituiert sein kann;
- A: ist bei jedem Auftreten gleich oder verschieden C(R¹)₂, NR¹, O oder S;
- n: ist 0 oder 1, wobei n = 0 bedeutet, dass an dieser Position keine Gruppe A gebunden ist und an den entsprechenden Kohlenstoffatomen statt dessen Reste R¹ gebunden sind;
- m: ist 0 oder 1, wobei m = 0 bedeutet, dass die Gruppe Ar¹ nicht vorhanden ist und dass die entsprechende aromatische bzw. heteroaromatische Gruppe direkt an Y bzw. ein Kohlenstoffatom des Grundgerüsts in Formel (1) bzw. in den bevorzugten Ausführungsformen bzw. an das Stickstoffatom in der Gruppe NAr₂ gebunden ist; mit der Maßgabe, dass m = 1 ist für die Strukturen (Ar-12), (Ar-17), (Ar-21), (Ar-25), (Ar-26), (Ar-30), (Ar-34), (Ar-38) und (Ar-39), wenn es sich bei diesen Gruppen um Ausführungsformen von R' oder Ar handelt.

Wenn die oben genannten Gruppen für R, R' bzw. Ar mehrere Gruppen A aufweisen, so kommen hierfür alle Kombinationen aus der Definition von A in Frage. Bevorzugte Ausführungsformen sind dann solche, in denen eine Gruppe A für NR¹ und die andere Gruppe A für C(R¹)₂ steht oder in denen beide Gruppen A für NR¹ stehen oder in denen beide Gruppen A für O stehen. In einer besonders bevorzugten Ausführungsform der Erfindung steht in Gruppen R, R' bzw. Ar, die mehrere Gruppen A aufweisen, mindestens eine Gruppe A für C(R¹)₂ oder für NR¹.

Wenn A für NR¹ steht, steht der Substituent R¹, der an das Stickstoffatom gebunden ist, bevorzugt für ein aromatisches oder heteroaromatisches Ringsystem mit 5 bis 24 aromatischen Ringatomen, welches auch durch einen oder mehrere Reste R² substituiert sein kann. In einer besonders bevorzugten Ausführungsform steht dieser Substituent R¹ gleich oder verschieden bei jedem Auftreten für ein aromatisches oder heteroaromatisches Ringsystem mit 6 bis 24 aromatischen Ringatomen, welches keine kondensierten Arylgruppen aufweist und welches keine kondensierten Heteroarylgruppen, in denen zwei oder mehr aromatische bzw. heteroaromatische 6-Ring-Gruppen direkt aneinander ankondensiert sind, aufweist, und welches jeweils auch durch einen oder mehrere Reste R² substituiert sein kann. Besonders bevorzugt sind Phenyl, Biphenyl, Terphenyl und Quaterphenyl mit Verknüpfungsmustern, wie vorne für Ar-1 bis Ar-11 aufgeführt, wobei diese Strukturen durch einen oder mehrere Reste R² substituiert sein können, bevorzugt aber unsubstituiert sind.

Wenn A für C(R¹)₂ steht, stehen die Substituenten R¹, die an dieses Kohlenstoffatom gebunden sind, bevorzugt gleich oder verschieden bei jedem Auftreten für eine lineare Alkylgruppe mit 1 bis 10 C-Atomen oder für eine verzweigte oder cyclische Alkylgruppe mit 3 bis 10 C-Atomen oder für ein aromatisches oder heteroaromatisches Ringsystem mit 5 bis 24 aromatischen Ringatomen, welches auch durch einen oder mehrere Reste R² substituiert sein kann. Ganz besonders bevorzugt steht R¹ für eine Methylgruppe oder für eine Phenylgruppe. Dabei können die Reste R¹ auch miteinander ein Ringsystem bilden, was zu einem Spirosystem führt.

Weitere geeignete Gruppen R, R' bzw. Ar sind Gruppen der Formel -Ar⁴-N(Ar²)(Ar³), wobei Ar², Ar³ und Ar⁴ gleich oder verschieden bei jedem Auftreten für ein aromatisches oder heteroaromatisches Ringsystem mit 5 bis 24 aromatischen Ringatomen stehen, welches jeweils mit einem oder mehreren Resten R¹ substituiert sein kann. Dabei beträgt die Gesamtzahl der aromatischen Ringatome von Ar², Ar³ und Ar⁴ maximal 60 und bevorzugt maximal 40.

Dabei können Ar⁴ und Ar² miteinander und/oder Ar² und Ar³ miteinander auch durch eine Gruppe ausgewählt aus C(R¹)₂, NR¹, O oder S verbunden sein. Bevorzugt erfolgt die Verknüpfung von Ar⁴ und Ar² miteinander bzw. von Ar² und Ar³ miteinander jeweils ortho zur Position der Verknüpfung mit dem Stickstoffatom. In einer weiteren Ausführungsform der Erfindung sind keine der Gruppen Ar², Ar³ bzw. Ar⁴ miteinander verbunden.

Bevorzugt ist Ar⁴ ein aromatisches oder heteroaromatisches Ringsystem mit 6 bis 24 aromatischen Ringatomen, bevorzugt mit 6 bis 12 aromatischen Ringatomen, das jeweils mit einem oder mehreren Resten R¹ substituiert sein kann. Besonders bevorzugt ist Ar⁴ ausgewählt aus der Gruppe bestehend aus ortho-, meta- oder para-Phenylen oder ortho-, meta- oder para-Biphenyl, welche jeweils durch einen oder mehrere Reste R¹ substituiert sein können, bevorzugt aber unsubstituiert sind. Ganz besonders bevorzugt ist Ar⁴ eine unsubstituierte Phenylengruppe. Dies gilt insbesondere, wenn Ar⁴ mit Ar² durch einen Einfachbindung verbunden ist.

Bevorzugt sind Ar² und Ar³ gleich oder verschieden bei jedem Auftreten ein aromatisches oder heteroaromatisches Ringsystem mit 6 bis 24 aromatischen Ringatomen, das jeweils mit einem oder mehreren Resten R¹ substituiert sein kann. Besonders bevorzugte Gruppen Ar² bzw. Ar³ sind gleich oder verschieden bei jedem Auftreten ausgewählt aus der Gruppe bestehend aus Benzol, ortho-, meta- oder para-Biphenyl, ortho-, meta-, para- oder verzweigtem Terphenyl, ortho-, meta-, para- oder verzweigtem Quaterphenyl, 1-, 2-, 3- oder 4-Fluorenyl, 1-, 2-, 3- oder 4-Spirobifluorenyl, 1- oder 2-Naphthyl, Indol, Benzofuran, Benzothiophen, 1-, 2-, 3- oder 4-Carbazol, 1-, 2-, 3- oder 4-Dibenzofuran, 1-, 2-, 3- oder 4-Dibenzothiophen, Indenocarbazol, Indolocarbazol, 2-, 3- oder 4-Pyridin, 2-, 4- oder 5-Pyrimidin, Pyrazin, Pyridazin, Triazin, Phenanthren, Triphenylen oder Kombinationen aus zwei, drei oder vier dieser Gruppen, welche jeweils mit einem oder mehreren Resten R¹ substituiert sein können. Besonders bevorzugt stehen Ar² und Ar³ gleich oder verschieden bei jedem Auftreten für ein aromatisches Ringsystem mit 6 bis 24 aromatischen Ringatomen, welches durch einen oder mehrere Reste R¹ substituiert sein kann, insbesondere ausgewählt aus den Gruppen bestehend aus Benzol, Biphenyl, insbesondere ortho-, meta- oder para-Biphenyl, Terphenyl, insbesondere ortho-, meta-, para- oder verzweigtem Terphenyl, Quaterphenyl, insbesondere ortho-, meta-, para- oder verzweigtem Quaterphenyl, Fluoren, insbesondere 1-, 2-, 3- oder 4-Fluoren, oder Spirobifluoren, insbesondere 1-, 2-, 3- oder 4-Spirobifluoren.

Dabei haben in erfindungsgemäßen Verbindungen, die durch Vakuumverdampfung verarbeitet werden, die Alkylgruppen bevorzugt nicht mehr als fünf C-Atome, besonders bevorzugt nicht mehr als 4 C-Atome, ganz besonders bevorzugt nicht mehr als 1 C-Atom. Für Verbindungen, die aus Lösung verarbeitet werden, eignen sich auch Verbindungen, die mit Alkylgruppen, insbesondere verzweigten Alkylgruppen, mit bis zu 10 C-Atomen substituiert sind oder die mit Oligoarylengruppen, beispielsweise ortho-, meta-, para- oder verzweigten Terphenyl- oder Quaterphenylgruppen, substituiert sind.

Wenn die Verbindungen der Formel (1) bzw. die bevorzugten Ausführungsformen als Matrixmaterial für einen phosphoreszierenden Emitter oder in einer Schicht, die direkt an eine phosphoreszierende Schicht angrenzt, verwendet werden, ist es weiterhin bevorzugt, wenn die Verbindung keine kondensierten Aryl- bzw. Heteroarylgruppen enthält, in denen mehr als zwei Sechsringe direkt aneinander ankondensiert sind. Insbesondere ist es bevorzugt, dass die Reste R, R', Ar, R¹ und R² keine kondensierten Aryl- bzw. Heteroarylgruppen enthalten, in denen zwei oder mehr Sechsringe direkt aneinander ankondensiert sind. Eine Ausnahme hiervon bilden Phenanthren und Triphenylen, die aufgrund ihrer hohen Triplettenergie trotz der Anwesenheit kondensierter aromatischer Sechsringe bevorzugt sein können.

Die oben genannten bevorzugten Ausführungsformen können beliebig innerhalb der in Anspruch 1 definierten Einschränkungen miteinander kombiniert werden. In einer besonders bevorzugten Ausführungsform der Erfindung treten die oben genannten Bevorzugungen gleichzeitig auf.

Beispiele für bevorzugte Verbindungen gemäß den oben aufgeführten Ausführungsformen sind die in der folgenden Tabelle aufgeführten Verbindungen.

| | | |
|---|---|---|
| 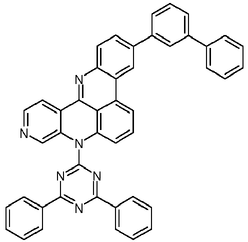 | 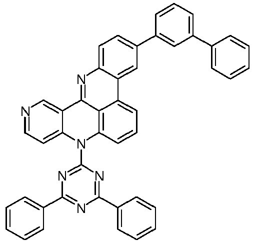 | 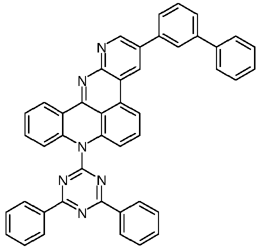 |
| 1 | 2 | 3 |
| 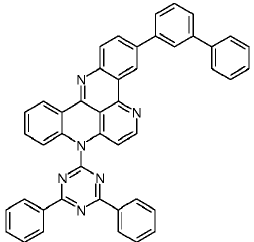 | 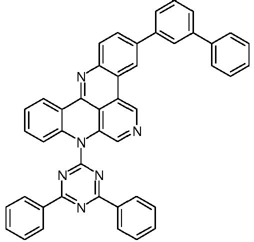 | 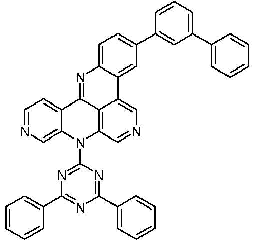 |
| 4 | 5 | 6 |
| 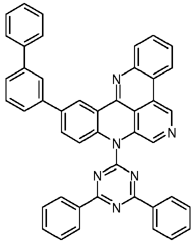 | 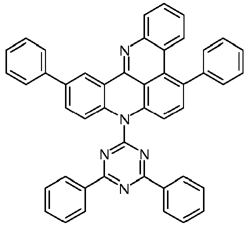 | 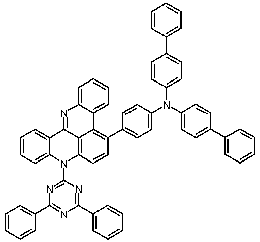 |
| 7 | 8 | 9 |
| 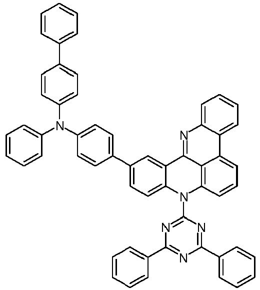 | 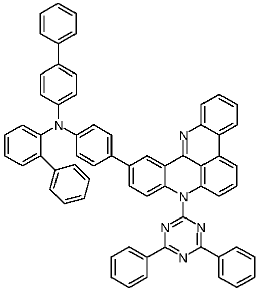 | 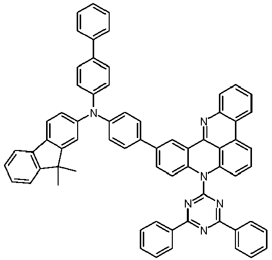 |
| 10 | 11 | 12 |
| 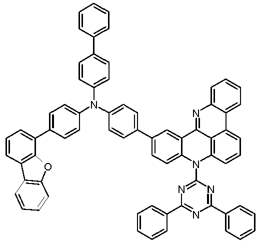 | 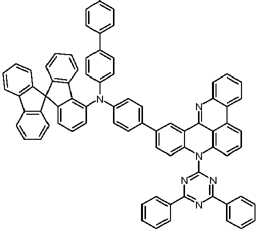 | 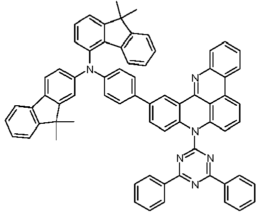 |
| 13 | 14 | 15 |
| 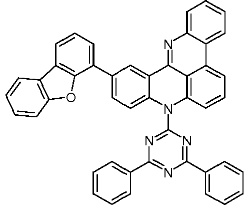 | 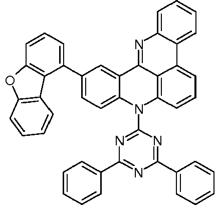 | 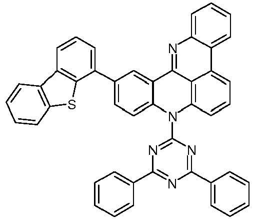 |
| 16 | 17 | 18 |
| 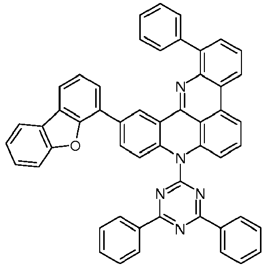 | 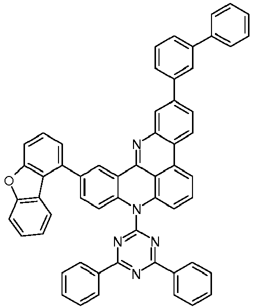 | 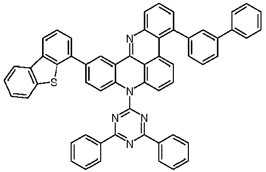 |
| 19 | 20 | 21 |
| 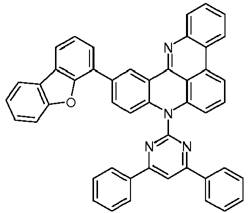 | 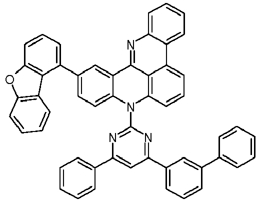 | 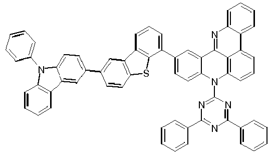 |
| 22 | 23 | 24 |
| 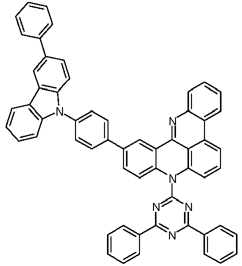 | 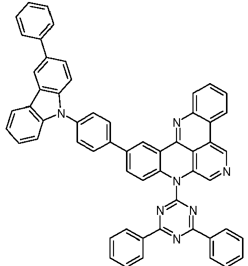 | 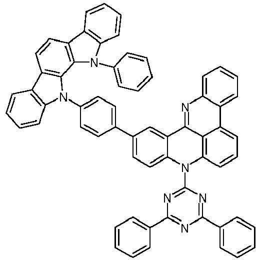 |
| 25 | 26 | 27 |
| 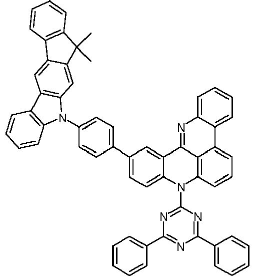 | 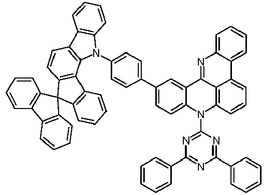 | 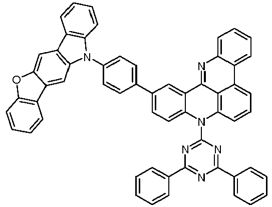 |
| 28 | 29 | 30 |
| 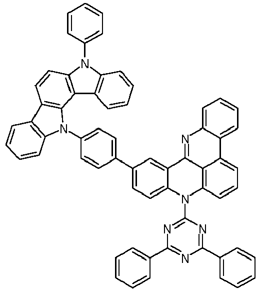 | 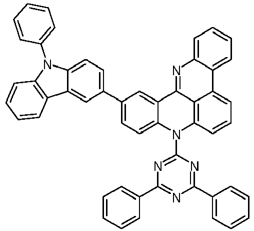 | 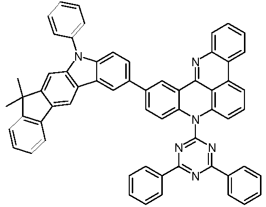 |
| 31 | 32 | 33 |
| 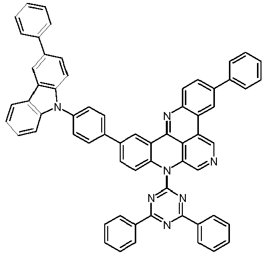 | 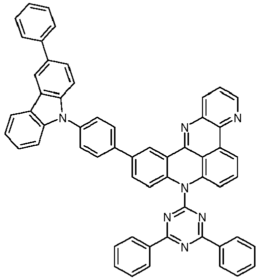 | 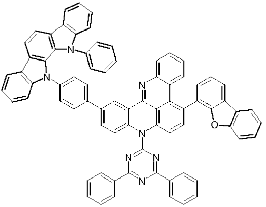 |
| 34 | 35 | 36 |
| 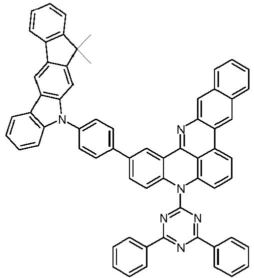 | 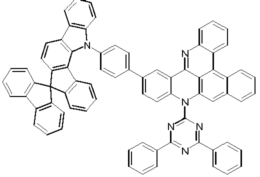 | 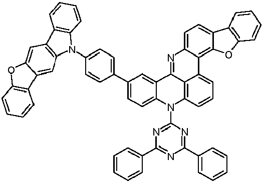 |
| 37 | 38 | 39 |
| 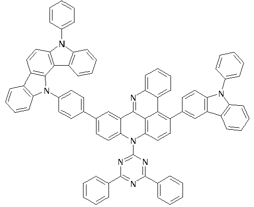 | 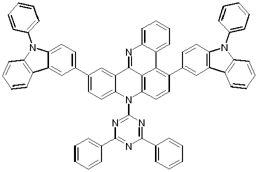 | 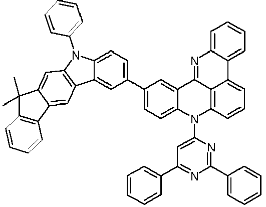 |
| 40 | 41 | 42 |
| 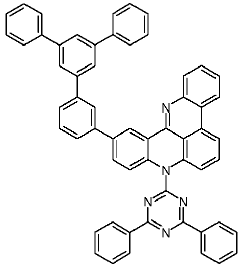 | 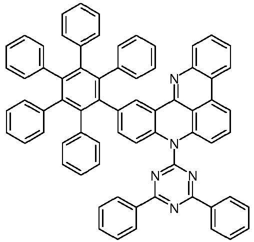 | 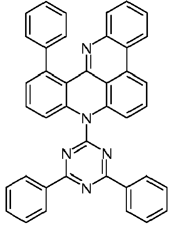 |
| 43 | 44 | 45 |
| 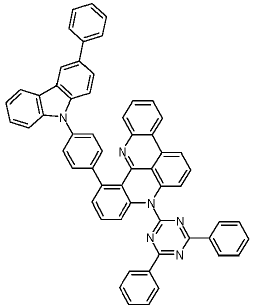 | 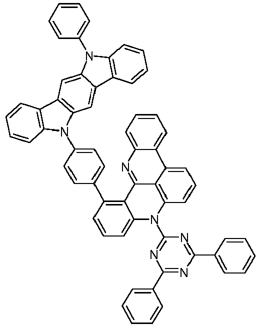 | 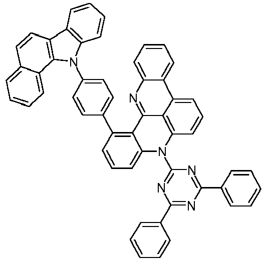 |
| 46 | 47 | 48 |
| 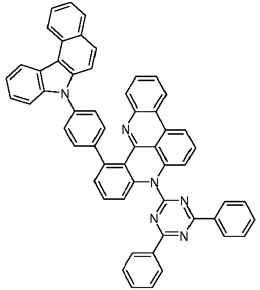 | 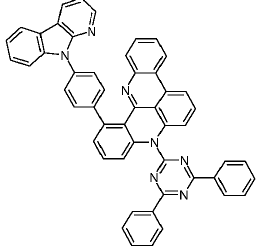 | 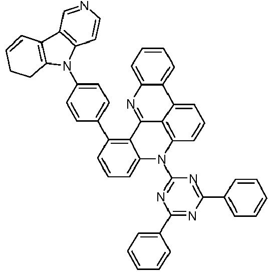 |
| 49 | 50 | 51 |
| 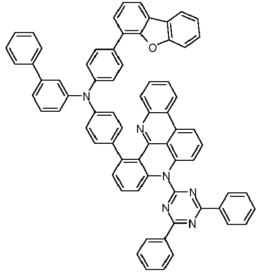 | 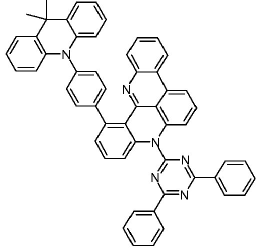 | 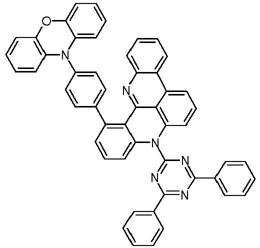 |
| 52 | 53 | 54 |
| 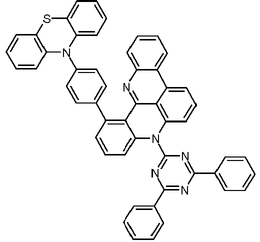 | 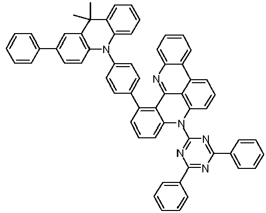 | 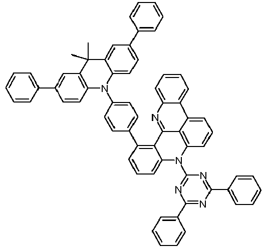 |
| 55 | 56 | 57 |
| 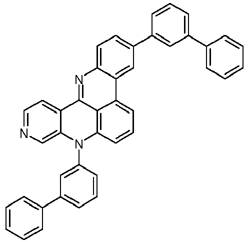 | 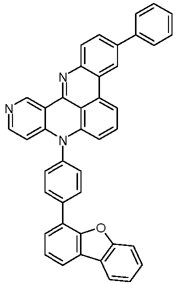 | 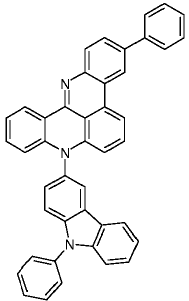 |
| 58 | 59 | 60 |
| 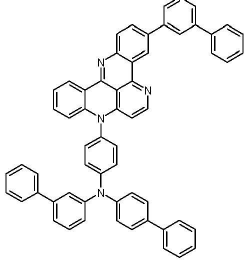 | 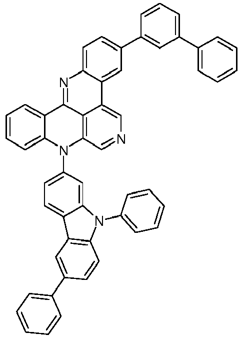 | 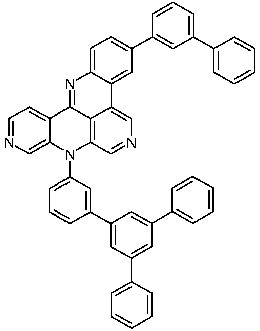 |
| 61 | 62 | 63 |
| 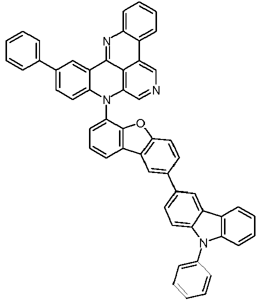 | 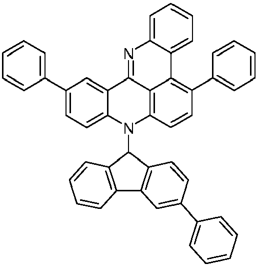 | 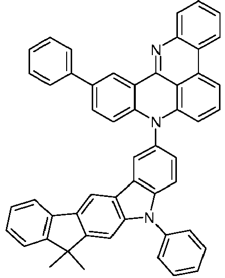 |
| 64 | 65 | 66 |
| 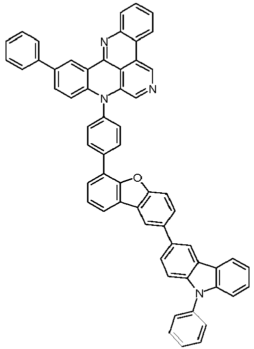 | 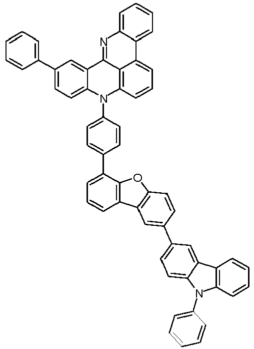 | 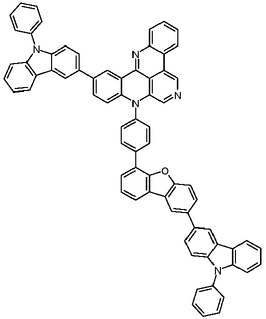 |
| 67 | 68 | 69 |
| 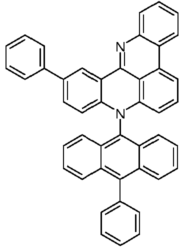 | 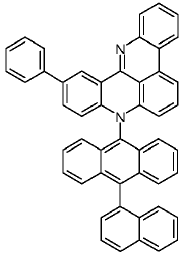 | 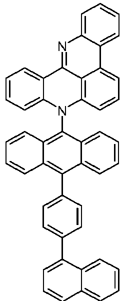 |
| 70 | 71 | 72 |
| 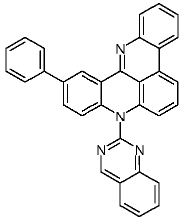 | 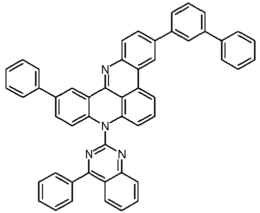 | 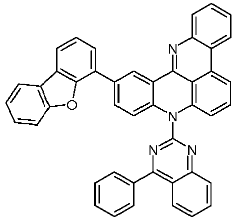 |
| 73 | 74 | 75 |
| 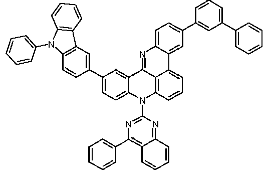 | 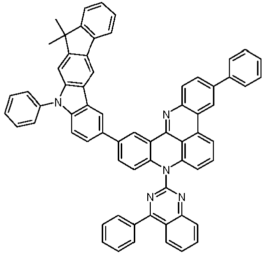 | 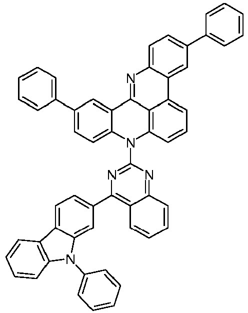 |
| 76 | 77 | 78 |
| 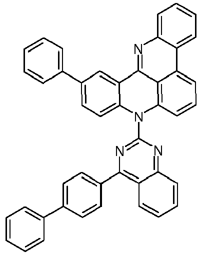 | 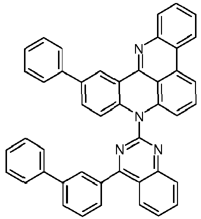 | 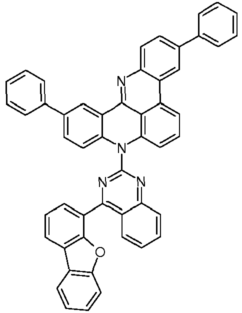 |
| 79 | 80 | 81 |
| 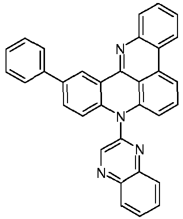 | 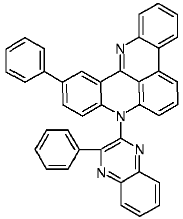 | 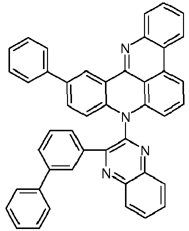 |
| 82 | 83 | 84 |
| 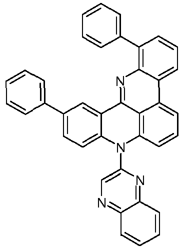 | 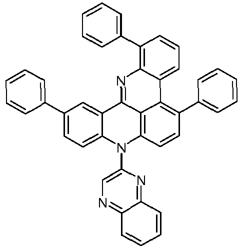 | 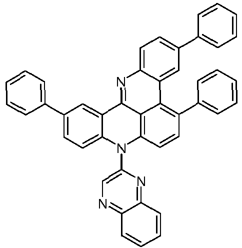 |
| 85 | 86 | 87 |
| 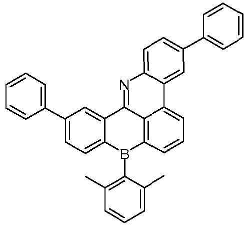 | 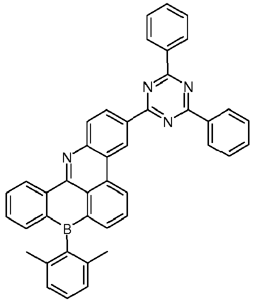 | 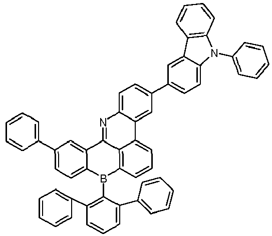 |
| 88 | 89 | 90 |
| 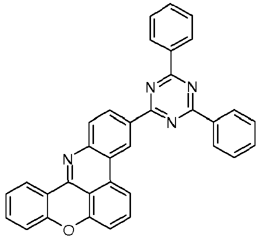 | 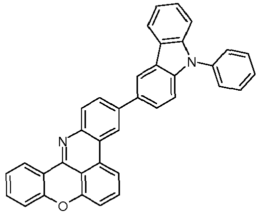 | 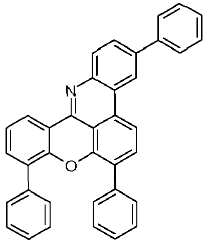 |
| 91 | 92 | 93 |
| 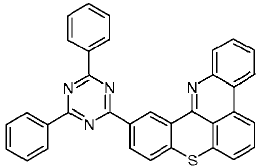 | 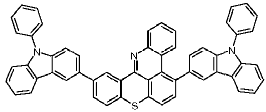 | 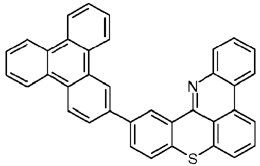 |
| 94 | 95 | 96 |
| 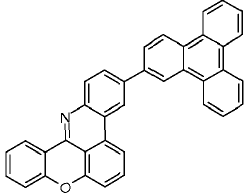 | 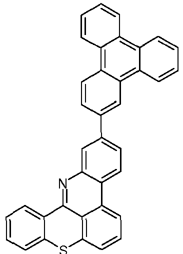 | 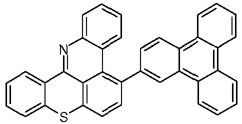 |
| 97 | 98 | 99 |
| 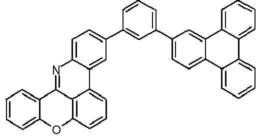 | 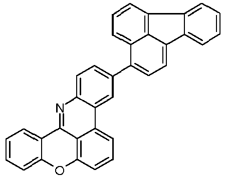 | 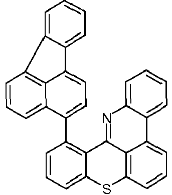 |
| 100 | 101 | 102 |
| 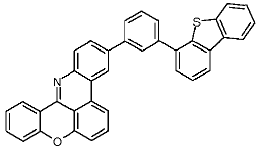 | 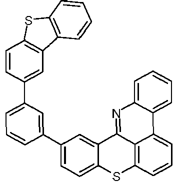 | 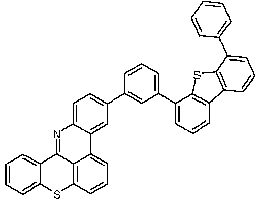 |
| 103 | 104 | 105 |
| 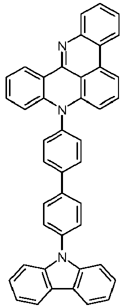 | 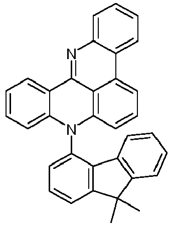 | 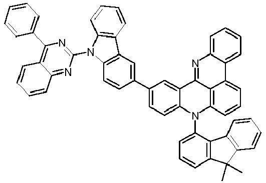 |
| 106 | 107 | 108 |
| 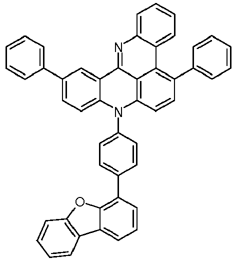 | | |
| 109 | | |

Die Synthese des unsubstituierten Grundgerüsts ist literaturbekannt und kann beispielsweise gemäß D. J. Hagan et al. (J. Chem. Soc., Perkin Transactions 1: Organic and Bio-Organic Chemistry, 1997, (18), 2739-2746) oder gemäß J. Stanslas et al. (J. Med. Chem., 2000, 43(8), 1563-1572) erfolgen.

Die erfindungsgemäßen Verbindungen können aus den 8H-Chino[4,3,2-kl] acridinen und halogenierten elektronenarmen Heterocyclen (Triazinen, Pyrimidinen, Pyridinen, Pyrazolen, Imidazolen, etc.) unter Einsatz einer starken Base (NaH, Na-/K-Alkoholate, Alkyl-/Aryllithiumverbindungen, etc.) dargestellt werden, wie im nachfolgenden Schema 1 gezeigt.

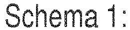

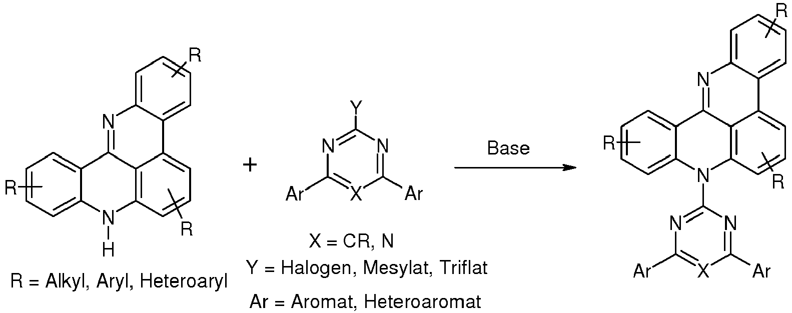

Die erfindungsgemäßen Verbindungen können auch aus den 8H-Chino[4, 3,2-kl]acridinen und halogenierten Aromaten-/Heteroaromaten durch Palladium- oder Kupfer-katalysierte Buchwald-Kupplung oder Ullmannanaloge Kupplung unter Einsatz einer Base dargestellt werden, wie im nachfolgenden Schema 2 gezeigt.

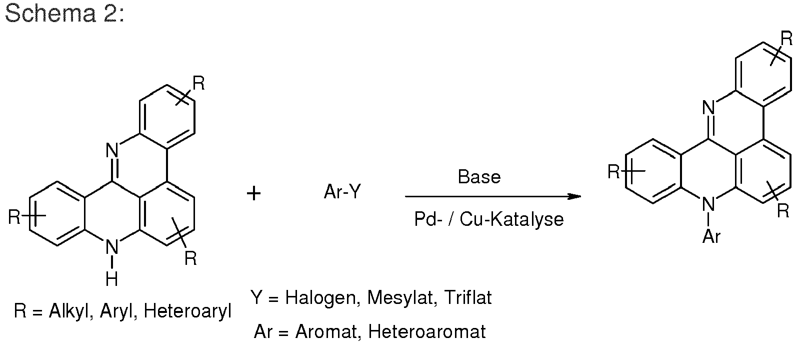

Halogen- bzw. Triflat-funktionalisierte 8H-Chino[4,3,2-kl]acridine, [1]Benzopyrano[4,3,2-gh]phenanthridine bzw. [1]Benzothiopyrano[4,3,2-gh]phenanthridine können nach dem Fachmann geläufigen Methoden, z. B. via Suzuki-Kupplung, weiter funktionalisiert werden, wie im nachfolgenden Schema 3 gezeigt.

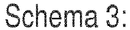

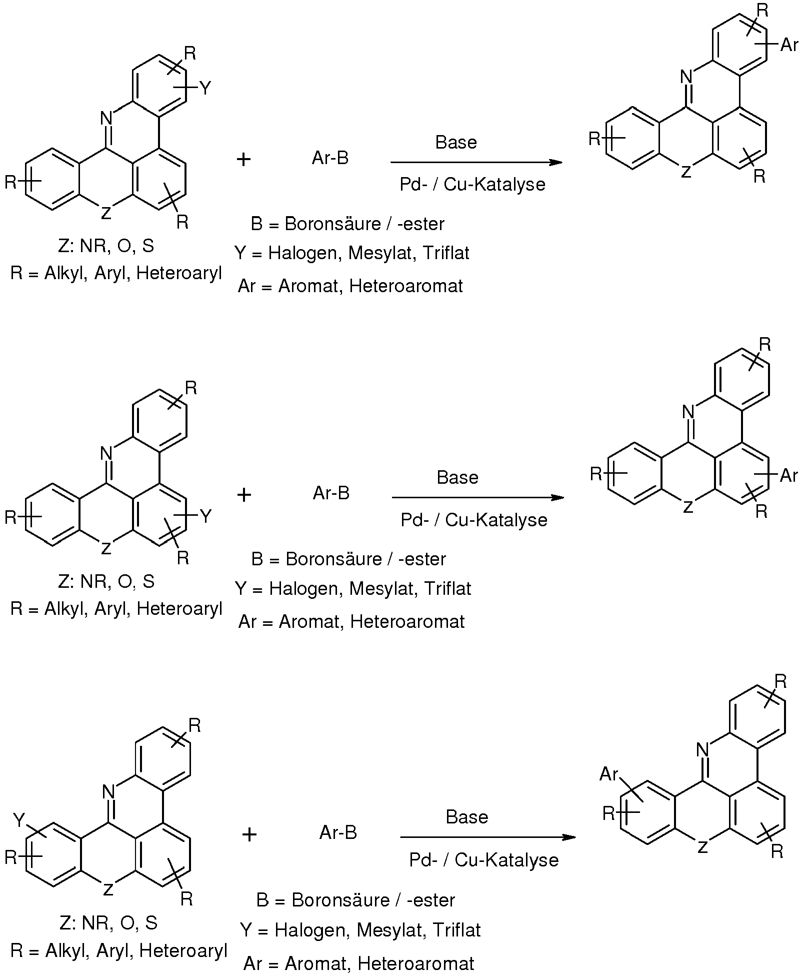

Halogen- bzw. Triflat-funktionalisierte 8H-Chino[4,3,2-kl]acridine, [1]Benzopyrano[4,3,2-gh]phenanthridine bzw. [1]Benzothiopyrano[4,3,2-gh]-phenanthridine können nach dem Fachmann geläufigen Methoden, z. B. via Buchwald- oder Ullmann-Kupplung, mit sekundären Aminen oder Carbazolen weiter funktionalisiert werden, wie in Schema 4 gezeigt.

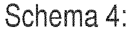

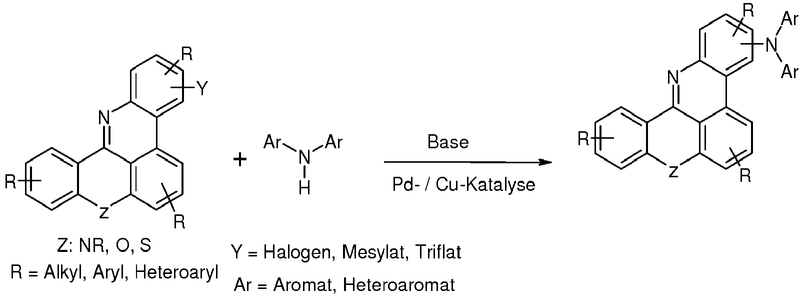

Es können selbstverständlich bei den o. g. Reaktionen ganz analog di-, tri- bzw. oligohalogen-funktionalisierte Edukte zum Einsatz kommen.

Ein weiterer Gegenstand der vorliegenden Erfindung ist ein Verfahren zur Herstellung einer Verbindung gemäß Formel (1) bzw. der bevorzugten Ausführungsformen, umfassend die Reaktionsschritte:
a) Bereitstellung des Grundgerüsts mit einer reaktiven Abgangsgruppe; und
b) Kupplung eines aromatischen bzw. heteroaromatischen Ringsystems bzw. einer Verbindung H-NAr₂ mit dem Grundgerüst unter Abspaltung der Abgangsgruppe.

Geeignete Abgangsgruppen sind beispielsweise Halogen, Triflat oder Mesylat, aber auch Wasserstoff, wenn die Kupplung in Schritt B an der Gruppe Y (für Y = NR') erfolgt.

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropyleneglycoldimethylether, Tetraethylenglycoldimethylether, 2-Isopropylnaphthalin, Pentylbenzol, Hexylbenzol, Heptylbenzol, Octylbenzol, 1,1-Bis(3,4-dimethylphenyl)ethan, 2-Methylbiphenyl, 3-Methylbiphenyl, 1-Methylnaphthalin, 1-Ethylnaphthalin, Ethyloctanoat, Sebacinsäure-diethylester, Octyloctanoat, Heptylbenzol, Menthyl-isovalerat, Cyclohexylhexanoat oder Mischungen dieser Lösemittel.

Ein weiterer Gegenstand der vorliegenden Erfindung ist daher eine Formulierung, enthaltend eine erfindungsgemäße Verbindung und mindestens eine weitere Verbindung. Die weitere Verbindung kann beispielsweise ein Lösemittel sein, insbesondere eines der oben genannten Lösemittel oder eine Mischung dieser Lösemittel. Die weitere Verbindung kann aber auch mindestens eine weitere organische oder anorganische Verbindung sein, die ebenfalls in der elektronischen Vorrichtung eingesetzt wird, beispielsweise eine emittierende Verbindung und/oder ein weiteres Matrix-material. Geeignete emittierende Verbindungen und weitere Matrixmaterialien sind hinten im Zusammenhang mit der organischen Elektrolumineszenzvorrichtung aufgeführt. Diese weitere Verbindung kann auch polymer sein.

Die erfindungsgemäßen Verbindungen eignen sich für die Verwendung in einer elektronischen Vorrichtung, insbesondere in einer organischen Elektrolumineszenzvorrichtung.

Ein weiterer Gegenstand der vorliegenden Erfindung ist daher die Verwendung einer erfindungsgemäßen Verbindung in einer elektronischen Vorrichtung, insbesondere in einer organischen Elektrolumineszenzvorrichtung.

Ein nochmals weiterer Gegenstand der vorliegenden Erfindung ist eine elektronische Vorrichtung enthaltend mindestens eine erfindungsgemäße Verbindung.

Eine elektronische Vorrichtung im Sinne der vorliegenden Erfindung ist eine Vorrichtung, welche mindestens eine Schicht enthält, die mindestens eine organische Verbindung enthält. Das Bauteil kann dabei auch anorganische Materialien enthalten oder auch Schichten, welche vollständig aus anorganischen Materialien aufgebaut sind.

Die elektronische Vorrichtung ist bevorzugt ausgewählt aus der Gruppe bestehend aus organischen Elektrolumineszenzvorrichtungen (OLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), farbstoffsensibilisierten organischen Solarzellen (DSSCs), organischen optischen Detektoren, organischen Photorezeptoren, organischen Feld-Quench-Devices (O-FQDs), lichtemittierenden elektrochemischen Zellen (LECs), organischen Laserdioden (O-Laser) und "organic plasmon emitting devices", bevorzugt aber organischen Elektrolumineszenzvorrichtungen (OLEDs), besonders bevorzugt phosphoreszierenden OLEDs.

Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten und/oder Ladungserzeugungsschichten (Charge-Generation Layers). Ebenso können zwischen zwei emittierende Schichten Interlayer eingebracht sein, welche beispielsweise eine exzitonenblockierende Funktion aufweisen. Es sei aber darauf hingewiesen, dass nicht notwendigerweise jede dieser Schichten vorhanden sein muss. Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Systeme mit drei emittierenden Schichten, wobei die drei Schichten blaue, grüne und orange oder rote Emission zeigen. Es kann sich bei der erfindungsgemäßen organischen Elektrolumineszenzvorrichtung auch um eine Tandem-OLED handeln, insbesondere für weiß emittierende OLEDs.

Die erfindungsgemäße Verbindung gemäß den oben aufgeführten Ausführungsformen kann dabei in unterschiedlichen Schichten eingesetzt werden, je nach genauer Struktur. Bevorzugt ist eine organische Elektrolumineszenzvorrichtung, enthaltend eine Verbindung gemäß Formel (1) bzw. die oben ausgeführten bevorzugten Ausführungsformen in einer emittierenden Schicht als Matrixmaterial für phosphoreszierende Emitter oder für Emitter, die TADF (thermally activated delayed fluorescence) zeigen, insbesondere für phosphoreszierende Emitter. Dabei kann die organische Elektrolumineszenzvorrichtung eine emittierende Schicht enthalten, oder sie kann mehrere emittierende Schichten enthalten, wobei mindestens eine emittierende Schicht mindestens eine erfindungsgemäße Verbindung als Matrixmaterial enthält. Weiterhin kann die erfindungsgemäße Verbindung auch in einer Elektronentransportschicht und/oder in einer Lochtransportschicht und/oder in einer Exzitonenblockierschicht und/oder in einer Lochblockierschicht eingesetzt werden. Insbesondere mit Y = O ist auch die Verwendung in einer Elektronentransportschicht bzw. Lochblockierschicht bevorzugt.

Wenn die erfindungsgemäße Verbindung als Matrixmaterial für eine phosphoreszierende Verbindung in einer emittierenden Schicht eingesetzt wird, wird sie bevorzugt in Kombination mit einem oder mehreren phosphoreszierenden Materialien (Triplettemitter) eingesetzt. Unter Phosphoreszenz im Sinne dieser Erfindung wird die Lumineszenz aus einem angeregten Zustand mit höherer Spinmultiplizität verstanden, also einem Spinzustand > 1, insbesondere aus einem angeregten Triplettzustand. Im Sinne dieser Anmeldung sollen alle lumineszierenden Komplexe mit Übergangsmetallen oder Lanthaniden, insbesondere alle Iridium-, Platin- und Kupferkomplexe als phosphoreszierende Verbindungen angesehen werden.

Die Mischung aus der erfindungsgemäßen Verbindung und der emittierenden Verbindung enthält zwischen 99 und 1 Vol.-%, vorzugsweise zwischen 98 und 10 Vol.-%, besonders bevorzugt zwischen 97 und 60 Vol.-%, insbesondere zwischen 95 und 80 Vol.-% der erfindungsgemäßen Verbindung bezogen auf die Gesamtmischung aus Emitter und Matrix-material. Entsprechend enthält die Mischung zwischen 1 und 99 Vol.-%, vorzugsweise zwischen 2 und 90 Vol.-%, besonders bevorzugt zwischen 3 und 40 Vol.-%, insbesondere zwischen 5 und 20 Vol.-% des Emitters bezogen auf die Gesamtmischung aus Emitter und Matrixmaterial.

Eine weitere bevorzugte Ausführungsform der vorliegenden Erfindung ist der Einsatz der erfindungsgemäßen Verbindung als Matrixmaterial für einen phosphoreszierenden Emitter in Kombination mit einem weiteren Matrixmaterial. Geeignete Matrixmaterialien, welche in Kombination mit den erfindungsgemäßen Verbindungen eingesetzt werden können, sind aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, Carbazolderivate, z. B. CBP (N,N-Biscarbazolylbiphenyl) oder die in WO 2005/039246, US 2005/0069729, JP 2004/288381, EP 1205527, WO 2008/086851 oder WO 2013/041176, Indolocarbazolderivate, z. B. gemäß WO 2007/063754 oder WO 2008/056746, Indenocarbazolderivate, z. B. gemäß WO 2010/136109, WO 2011/000455, WO 2013/041176 oder WO 2013/056776, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2007/063754, WO 2008/056746, WO 2010/015306, WO 2011/057706, WO 2011/060859 oder WO 2011/060877, Zinkkomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, verbrückte Carbazol-Derivate, z. B. gemäß WO 2011/042107, WO 2011/060867, WO 2011/088877 und WO 2012/143080, Triphenylenderivate, z. B. gemäß WO 2012/048781, oder Dibenzofuranderivate, z. B. gemäß WO 2015/169412, WO 2016/015810, WO 2016/023608 oder den noch nicht offen gelegten Anmeldungen EP 16158460.2 oder EP 16159829.7. Ebenso kann ein weiterer phosphoreszierender Emitter, welcher kürzerwellig als der eigentliche Emitter emittiert, als Co-Host in der Mischung vorhanden sein oder eine Verbindung, die nicht oder nicht in wesentlichem Umfang am Ladungstransport teilnimmt, wie beispielsweise in WO 2010/108579 beschrieben.

Insbesondere eignen sich in Kombination mit der erfindungsgemäßen Verbindung als Co-Matrix-Material Verbindungen, welche eine große Bandlücke aufweisen und selber nicht oder zumindest nicht in wesentlichem Maße am Ladungstransport der emittierenden Schicht teilnehmen. Es handelt sich bei solchen Materialien bevorzugt um reine Kohlenwasserstoffe. Beispiele für solche Materialien finden sich beispielsweise in der WO 2009/124627 oder in der WO 2010/006680.

Als phosphoreszierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten, insbesondere ein Metall mit dieser Ordnungszahl. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium oder Platin enthalten.

Beispiele der oben beschriebenen Emitter können den Anmeldungen WO 00/70655, WO 2001/41512, WO 2002/02714, WO 2002/15645, EP 1191613, EP 1191612, EP 1191614, WO 05/033244, WO 05/019373, US 2005/0258742, WO 2009/146770, WO 2010/015307, WO 2010/031485, WO 2010/054731, WO 2010/054728, WO 2010/086089, WO 2010/099852, WO 2010/102709, WO 2011/032626, WO 2011/066898, WO 2011/157339, WO 2012/007086, WO 2014/008982, WO 2014/023377, WO 2014/094961, WO 2014/094960, WO 2015/036074, WO 2015/104045, WO 2015/117718, WO 2016/015815, WO 2016/124304, WO 2017/032439 und der noch nicht offen gelegten Anmeldung EP 16179378.1 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenz bekannt sind, und der Fachmann kann ohne erfinderisches Zutun weitere phosphoreszierende Komplexe verwenden.

Explizite Beispiele für phosphoreszierende Dotanden sind in der folgenden Tabelle aufgeführt.

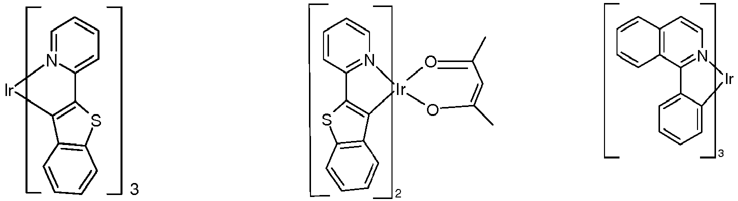

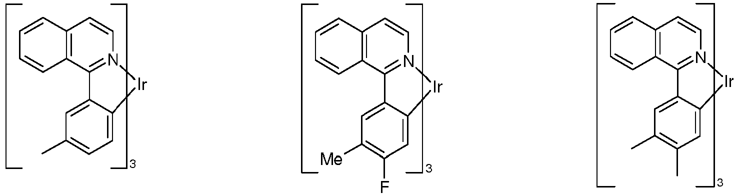

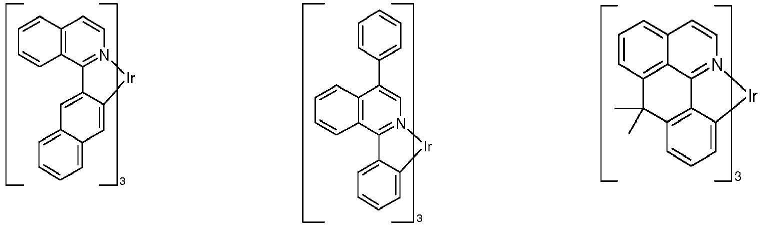

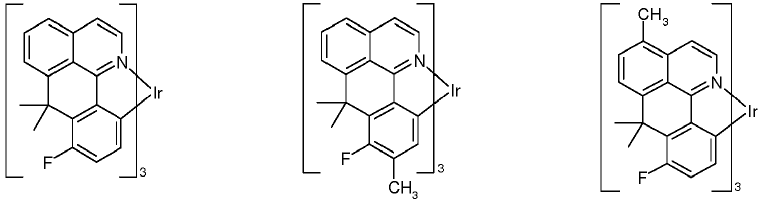

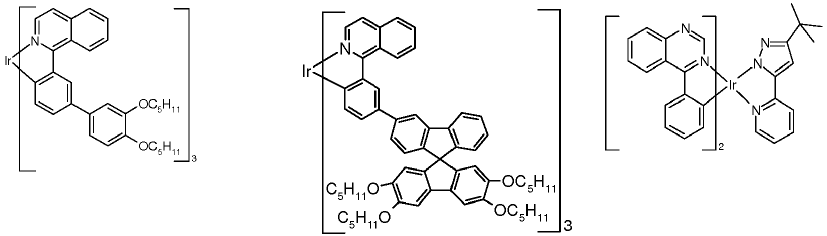

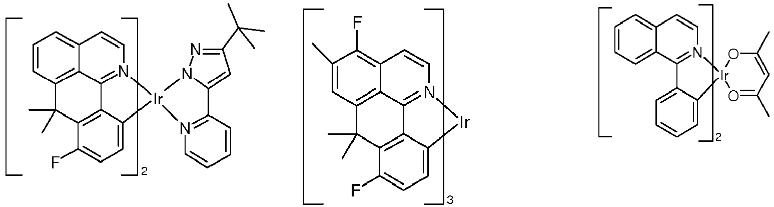

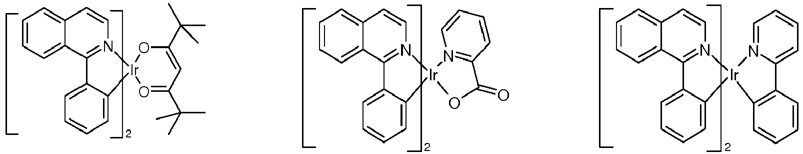

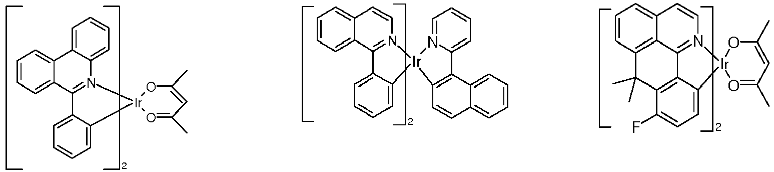

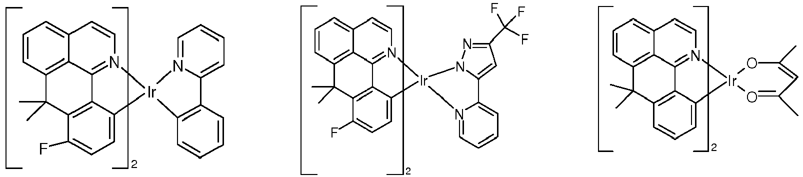

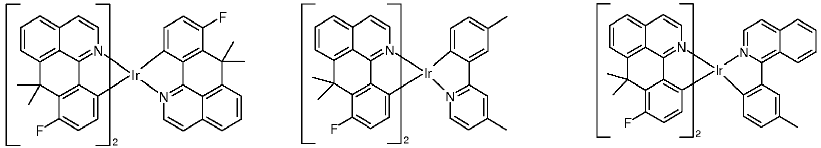

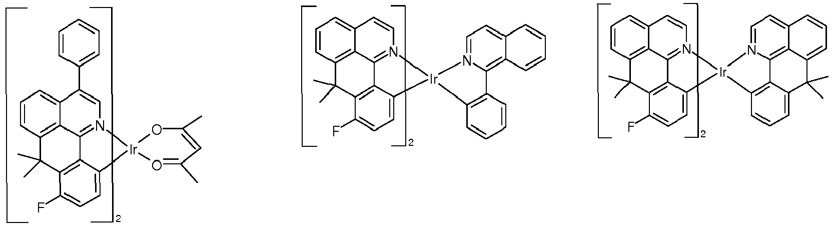

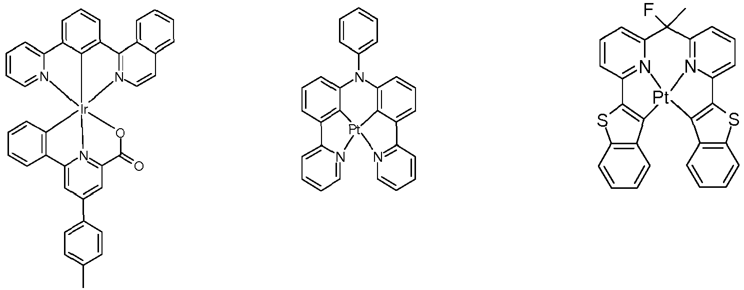

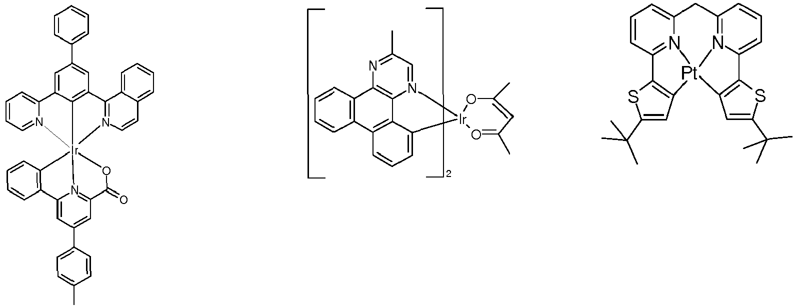

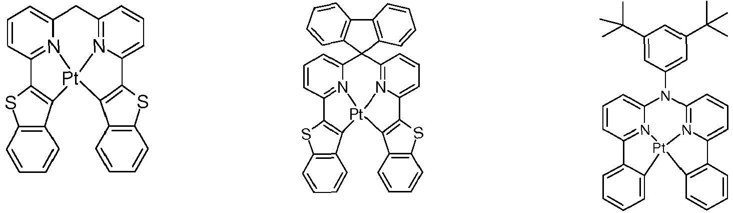

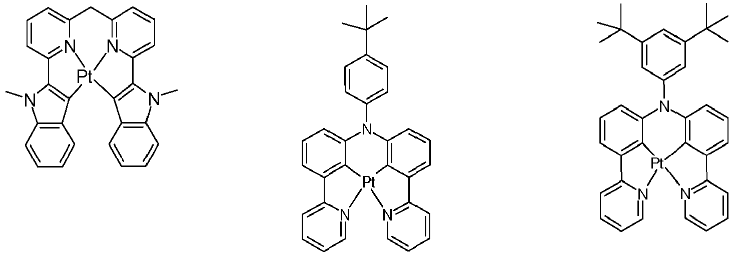

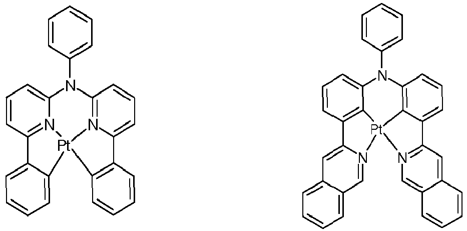

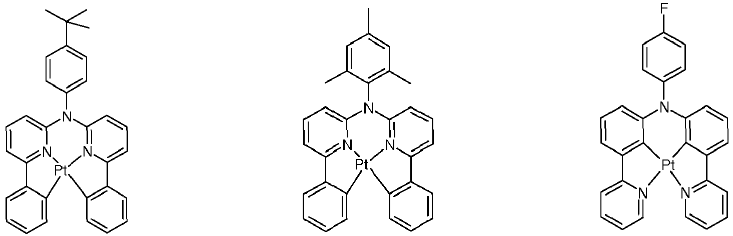

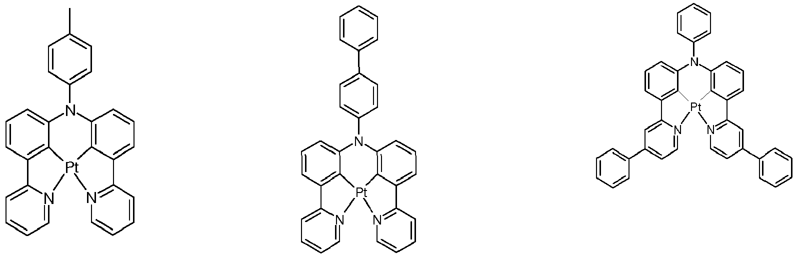

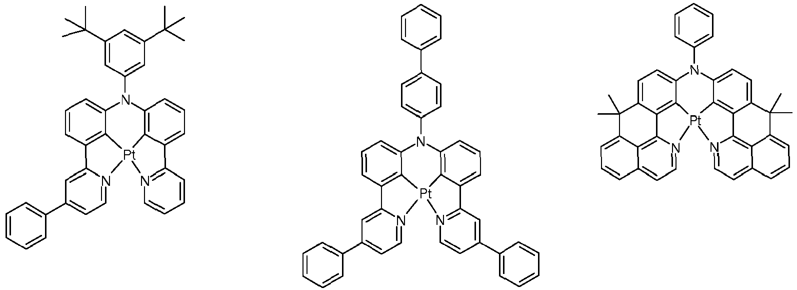

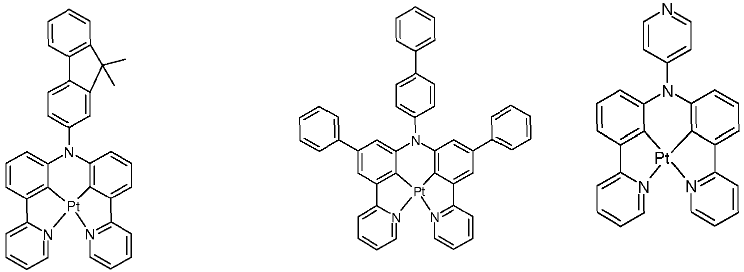

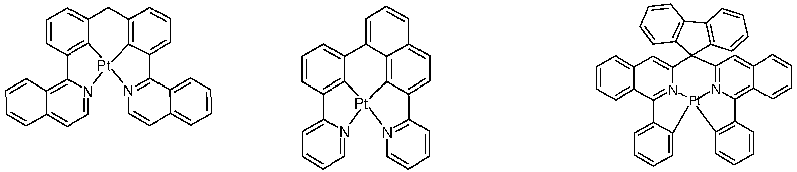

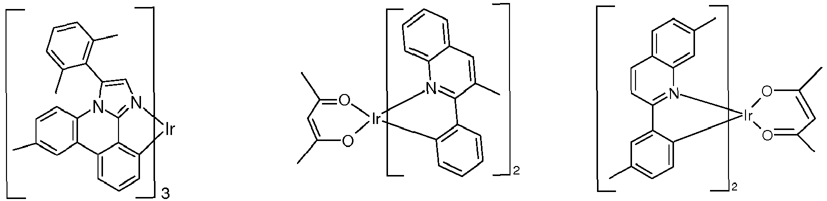

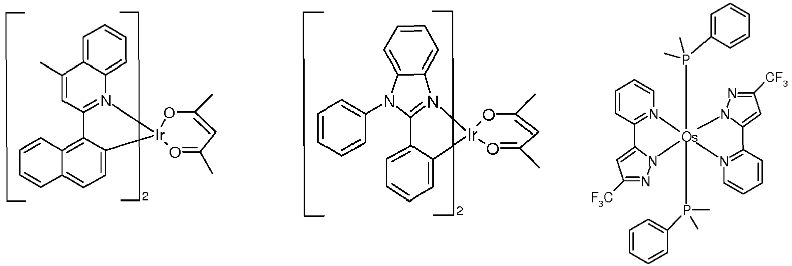

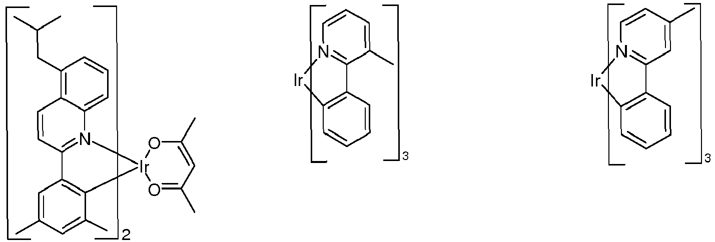

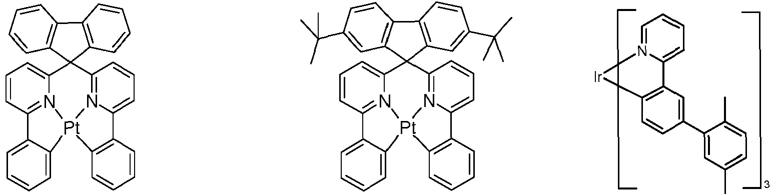

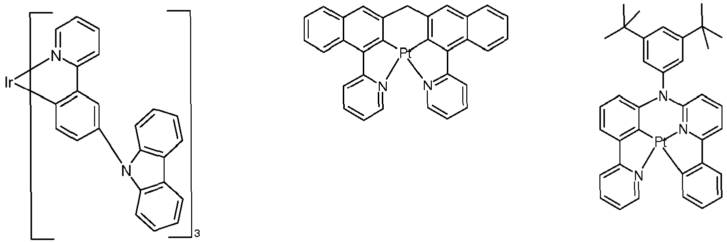

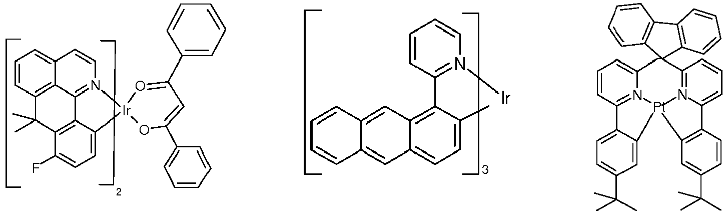

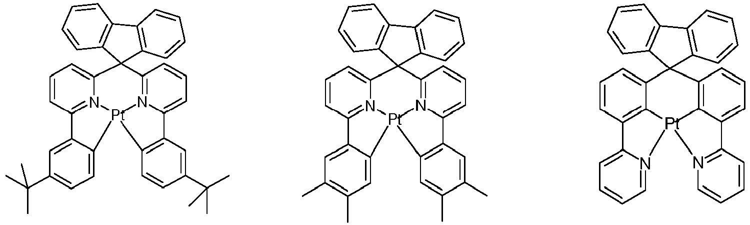

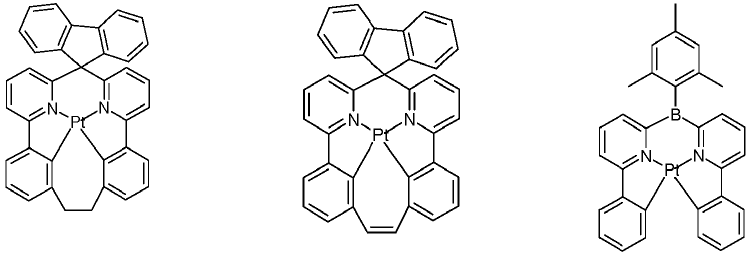

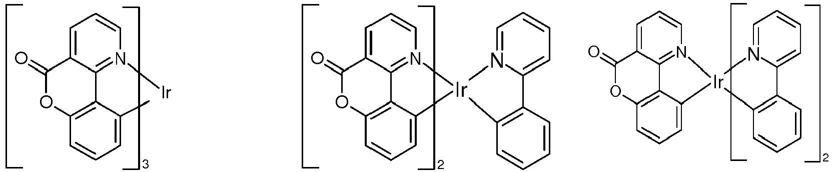

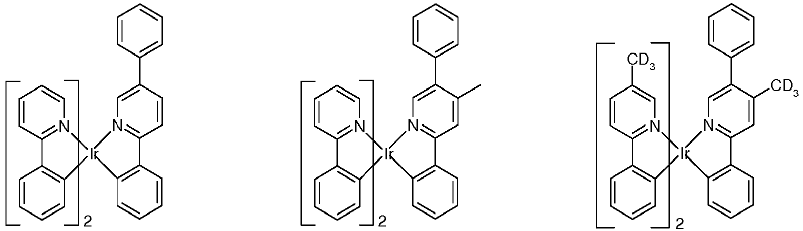

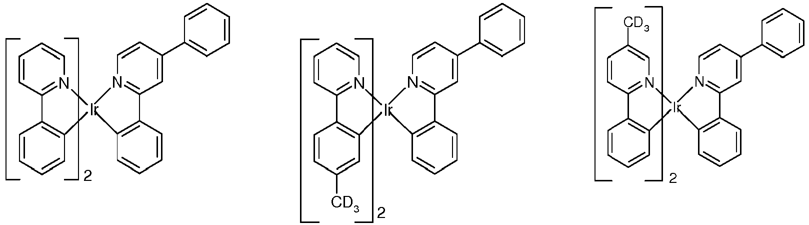

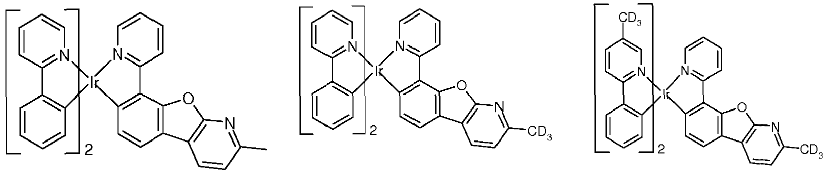

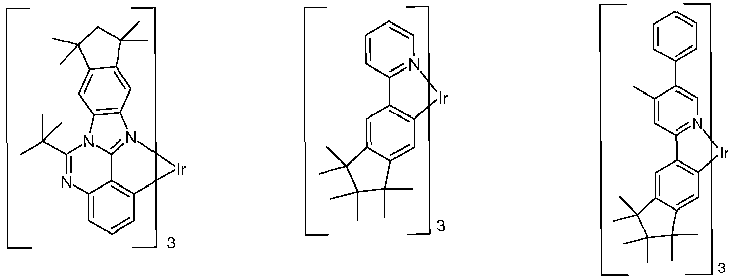

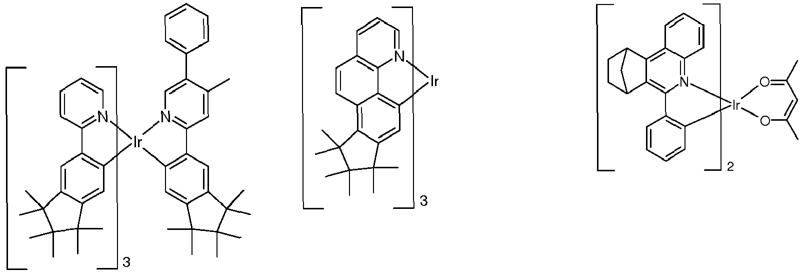

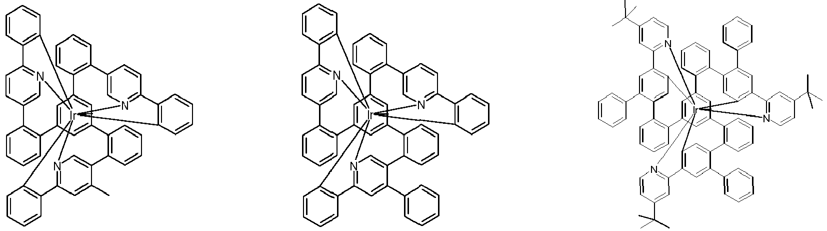

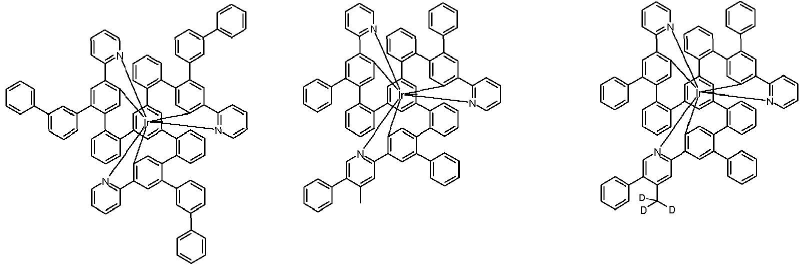

Die erfindungsgemäßen Verbindungen sind insbesondere auch geeignet als Matrixmaterialien für phosphoreszierende Emitter in organischen Elektrolumineszenzvorrichtungen, wie sie z. B. in WO 98/24271, US 2011/0248247 und US 2012/0223633 beschrieben sind. In diesen mehrfarbigen Display-Bauteilen wird eine zusätzliche blaue Emissionsschicht vollflächig auf alle Pixel, auch diejenigen mit einer von Blau verschiedenen Farbe, aufgedampft. Dabei wurde überraschend gefunden, dass die erfindungsgemäßen Verbindungen, wenn sie als Matrixmaterialien für das rote und/oder grüne Pixel eingesetzt werden, zusammen mit der aufgedampften blauen Emissionsschicht zu weiterhin sehr guter Emission führen.

In einer weiteren Ausführungsform der Erfindung enthält die erfindungsgemäße organische Elektrolumineszenzvorrichtung keine separate Lochinjektionsschicht und/oder Lochtransportschicht und/oder Lochblockierschicht und/oder Elektronentransportschicht, d. h. die emittierende Schicht grenzt direkt an die Lochinjektionschicht oder die Anode an, und/oder die emittierende Schicht grenzt direkt an die Elektronentransportschicht oder die Elektroneninjektionsschicht oder die Kathode an, wie zum Beispiel in WO 2005/053051 beschrieben. Weiterhin ist es möglich, einen Metallkomplex, der gleich oder ähnlich dem Metallkomplex in der emittierenden Schicht ist, direkt angrenzend an die emittierende Schicht als Lochtransport- bzw. Lochinjektionsmaterial zu verwenden, wie z. B. in WO 2009/030981 beschrieben.

In einer weiteren Ausführungsform der Erfindung wird die erfindungsgemäße Verbindung in einer Lochblockierschicht bzw. einer Elektronentransportschicht eingesetzt. Hierfür eignen sich insbesondere Verbindungen, in denen Y = O ist und/oder in welchen mindestens ein Symbol X für N steht und/oder welche mindestens einen Substituenten R oder R' aufweisen, der für eine elektronenarme Heteroarylgruppe steht bzw. eine elektronenarme Heteroarylgruppe enthält, beispielsweise Triazin oder Pyrimidin.

In den weiteren Schichten der erfindungsgemäßen organischen Elektrolumineszenzvorrichtung können alle Materialien verwendet werden, wie sie üblicherweise gemäß dem Stand der Technik eingesetzt werden. Der Fachmann kann daher ohne erfinderisches Zutun alle für organische Elektrolumineszenzvorrichtungen bekannten Materialien in Kombination mit den erfindungsgemäßen Verbindungen gemäß Formel (1) bzw. den oben ausgeführten bevorzugten Ausführungsformen einsetzen.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Es ist aber auch möglich, dass der Anfangsdruck noch geringer ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden.

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Offsetdruck, LITI (Light Induced Thermal Imaging, Thermotransferdruck), Ink-Jet Druck (Tintenstrahldruck) oder Nozzle Printing, hergestellt werden. Hierfür sind lösliche Verbindungen nötig, welche beispielsweise durch geeignete Substitution erhalten werden.

Weiterhin sind Hybridverfahren möglich, bei denen beispielsweise eine oder mehrere Schichten aus Lösung aufgebracht werden und eine oder mehrere weitere Schichten aufgedampft werden.

Diese Verfahren sind dem Fachmann generell bekannt und können von ihm ohne erfinderisches Zutun auf organische Elektrolumineszenzvorrichtungen enthaltend die erfindungsgemäßen Verbindungen angewandt werden.

Die erfindungsgemäßen Verbindungen und die erfindungsgemäßen organischen Elektrolumineszenzvorrichtungen zeichnen sich durch einen oder mehrere der folgenden überraschenden Vorteile gegenüber dem Stand der Technik aus:
1. Die erfindungsgemäßen Verbindungen, eingesetzt als Matrixmaterial für phosphoreszierende Emitter, führen zu langen Lebensdauern.
2. Die erfindungsgemäßen Verbindungen führen zu hohen Effizienzen. Dies gilt insbesondere, wenn die Verbindungen als Matrixmaterial für einen phosphoreszierenden Emitter eingesetzt werden.
3. Die erfindungsgemäßen Verbindungen führen zu geringen Betriebsspannungen. Dies gilt insbesondere, wenn die Verbindungen als Matrixmaterial für einen phosphoreszierenden Emitter eingesetzt werden.
4. Die erfindungsgemäße Verbindungen, insbesondere Verbindungen, in denen Y = O ist und/oder in welchen mindestens ein Symbol X für N steht und/oder welche mindestens einen elektronenarmen heteroaromatischen Substituenten aufweisen, führen bei Einsatz in einer Lochblockierschicht bzw. einer Elektronentransportschicht zu sehr guten Ergebnissen.

Diese oben genannten Vorteile gehen nicht mit einer Verschlechterung der weiteren elektronischen Eigenschaften einher.

Die Erfindung wird durch die nachfolgenden Beispiele näher erläutert, ohne sie dadurch einschränken zu wollen. Der Fachmann kann aus den Schilderungen die Erfindung im gesamten offenbarten Bereich ausführen und ohne erfinderisches Zutun weitere erfindungsgemäße Verbindungen herstellen und diese in elektronischen Vorrichtungen verwenden bzw. das erfindungsgemäße Verfahren anwenden.

### Beispiele:

Die nachfolgenden Synthesen werden, sofern nicht anders angegeben, unter einer Schutzgasatmosphäre in getrockneten Lösungsmitteln durchgeführt. Die Metallkomplexe werden zusätzlich unter Ausschluss von Licht bzw. unter Gelblicht gehandhabt. Die Lösungsmittel und Reagenzien können z. B. von Sigma-ALDRICH bzw. ABCR bezogen werden. Die jeweiligen Angaben in eckigen Klammern bzw. die zu einzelnen Verbindungen angegebenen Nummern beziehen sich auf die CAS-Nummern der literaturbekannten Verbindungen.

### Synthone und erfindungsgemäße Beispiele S:

### Beispiel S1

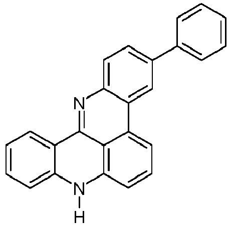

Ein Gemisch aus 30.3 g (100 mmol) 3-Chlor-8**H**-quino[4,3,2-**kl**]acridin [198025-90-0], 13.4 g (110 mmol) Phenylboronsäure [98-80-6], 69.1 g (300 mmol) Trikaliumphosphoat Monohydrat, 821 mg (2 mmol) SPhos [657408-07-6], 225 (1 mmol) Palladiumacetat, 50 g Glaskugeln (3 mm Durchmesser) und 300 ml DMSO wird 24 h auf 120 °C e rhitzt. Nach Erkalten entfernt man das DMSO weitgehend im Vakuum, nimmt den Rückstand in 500 ml Dichlormethan (DCM) auf, wäscht die organische Phase dreimal mit je 300 ml Wasser, einmal mit 300 ml gesättigter Kochsalzlösung und trocknet über Magnesiumsulfat. Man filtriert vom Trockenmittel über ein Celite-Bett ab und engt das Filtrat zur Trockene ein. Das Rohprodukt wird aus Dimethylacetamid umkristallisiert. Ausbeute: 24.8 g, (72 mmol), 72%; Reinheit: > 98%ig nach HPLC.

Analog können folgende Verbindungen dragestellt werden:

| Bsp. | Edukte | Produkt | Ausbeute |
|---|---|---|---|
| S2 | 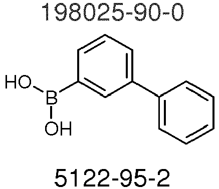 | 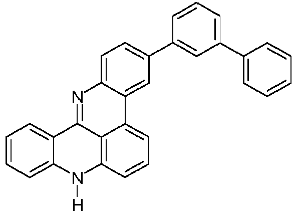 | 68 % |
| S3 | 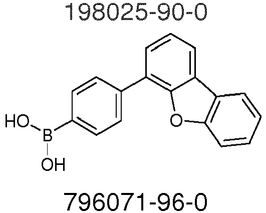 | 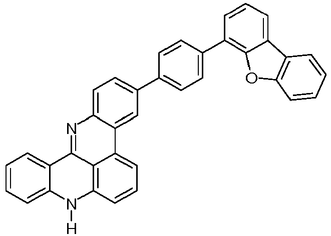 | 70 % |
| S4 | 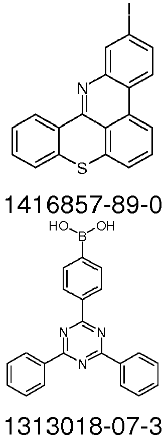 | 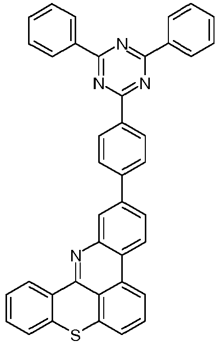 | 49 % |
| | | Umkristallisation aus DMAC Fraktionierte Sublimation p ca. 10⁻⁵ mbar / T ca. 320 °C | |
| S5 | 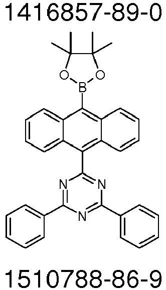 | 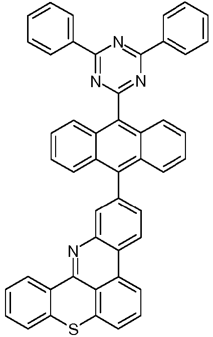 | 52 % |
| | | Umkristallisation aus DMAC Fraktionierte Sublimation p ca. 10⁻⁵ mbar / T ca. 330 °C | |
| S6 | 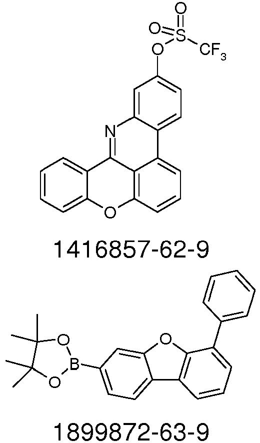 | 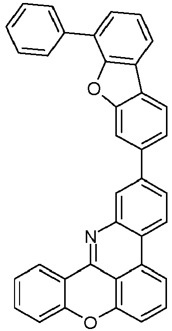 | 55 % |
| | | Umkristallisation aus DMF Fraktionierte Sublimation p ca. 10⁻⁵ mbar / T ca. 330 °C | |
| S7 | 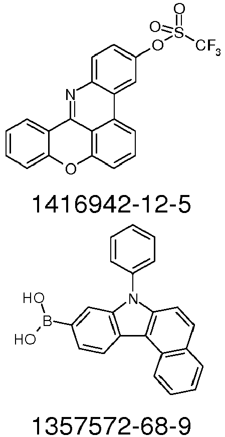 | 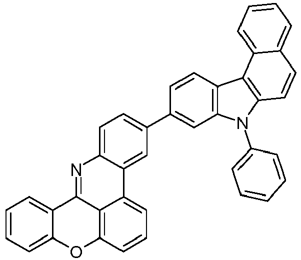 | 47% |
| | | Umkristallisation aus DMF Fraktionierte Sublimation p ca. 10⁻⁵ mbar / T ca. 330 °C | |

### Beispiel 1:

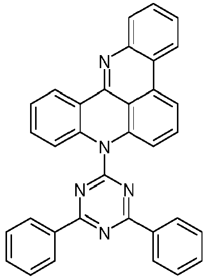

Eine Lösung von 26.8 g (100 mmol) 8**H**-Chino[4,3,2-**kl**]acridin [111180-95-5] und 26.8 g (100 mmol) Chlordiphenyltriazin [3842-55-5] in 300 ml Dimethylacetamid (DMAC) wird bei Raumtemperatur portionsweise mit 2.4 g (100 mmol) Natriumhydrid versetzt (Vorsicht: Wasserstoffentwicklung!). Nach beendeter Zugabe erwärmt man langsam auf 140 °C und rührt nach, bis die Edukte verbraucht sind (ca. 2 h). Nach Erkalten saugt man vom gelben nadligen Feststoff ab, wäscht diesen einmal mit 100 ml DMAC und dreimal mit je 100 ml Methanol und trocknet dann im Vakuum. Das so erhaltene Rohprpodukt wird fünfmal aus DMAC umkristallisiert und anschließend zweimal fraktioniert sublimiert (p ca. 10⁻⁵ mbar, T ca. 320 °C). Ausbeute: 26.0 g, (52 mmol), 52%; Reinheit: > 99.99 %ig nach HPLC.

Analog können folgende Verbindungen dargestellt werden:

| Bsp. | Edukte | Produkt | Ausbeute |
|---|---|---|---|
| 2 | 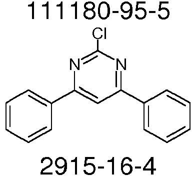 | 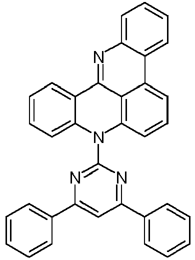 | 56 % |
| 3 | 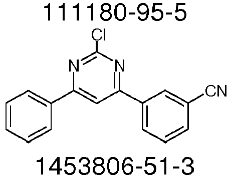 | 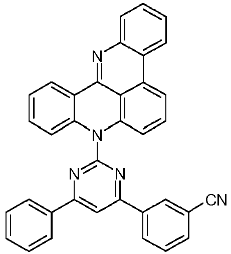 | 49 % |
| 4 | 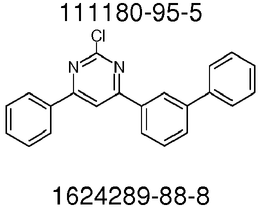 | 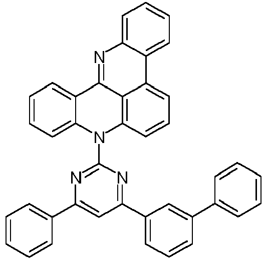 | 55 % |
| 5 | 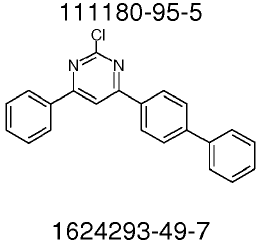 | 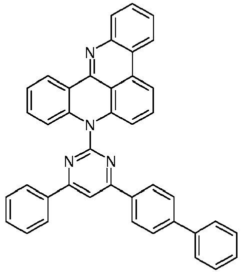 | 61 % |
| 6 | 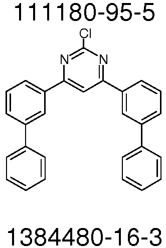 | 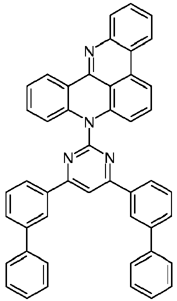 | 57 % |
| 7 | 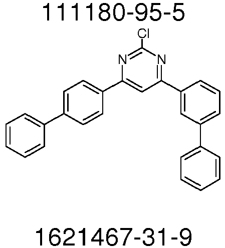 | 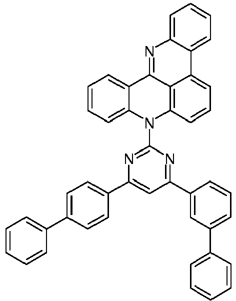 | 53 % |
| 8 | 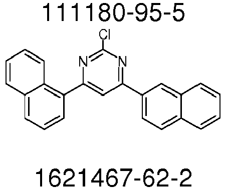 | 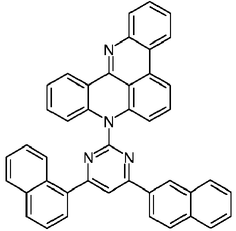 | 60 % |
| 9 | 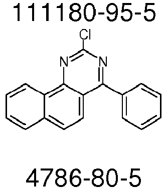 | 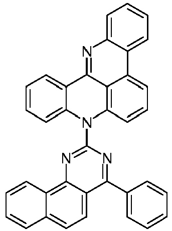 | 64 % |
| 10 | 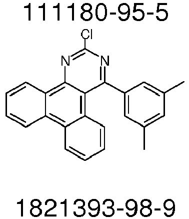 | 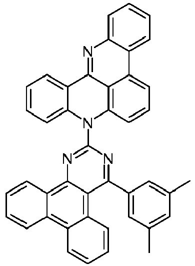 | 58 % |
| 11 | 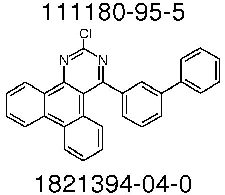 | 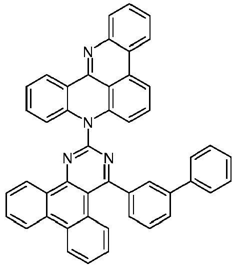 | 55 % |
| 12 | 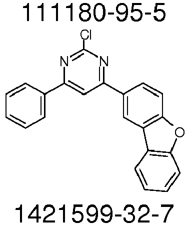 | 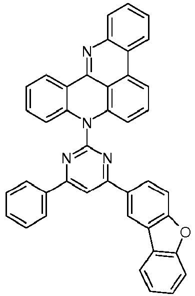 | 61 % |
| 13 | 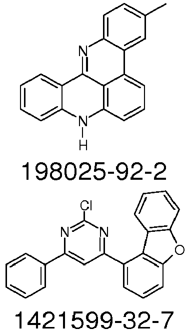 | 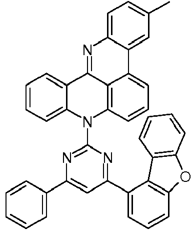 | 52 % |
| 14 | 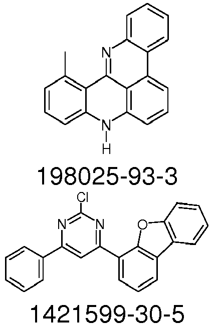 | 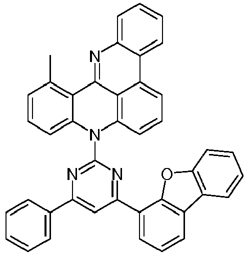 | 56 % |
| 15 | 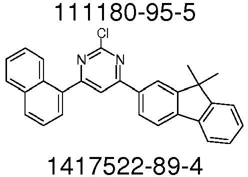 | 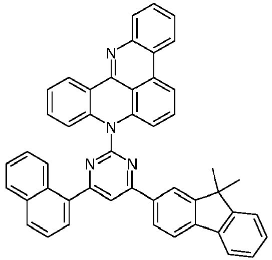 | 56 % |
| 16 | 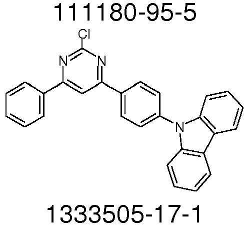 | 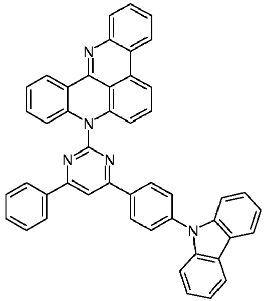 | 59 % |
| 17 | 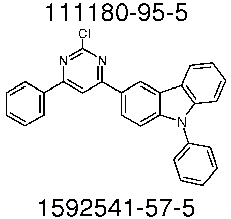 | 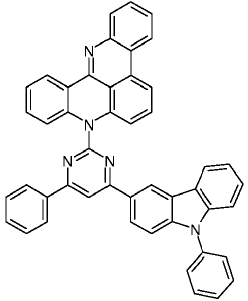 | 60 % |
| 18 | 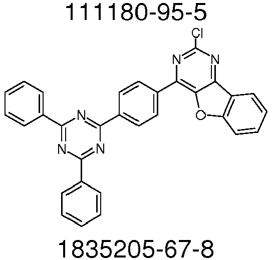 | 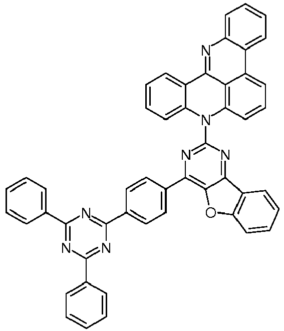 | 54 % |
| 19 | 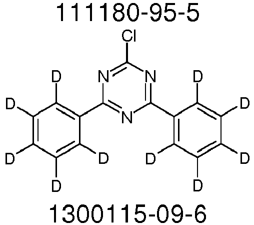 | 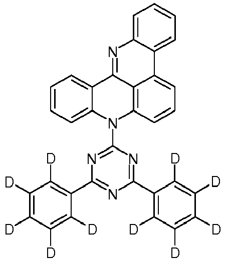 | 62 % |
| 20 | 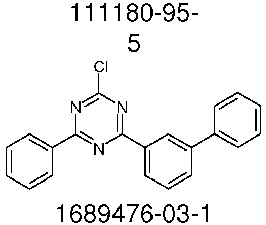 | 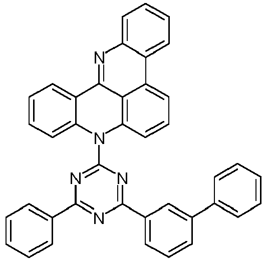 | 59 % |
| 21 | 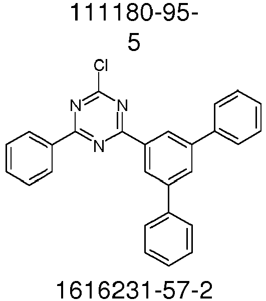 | 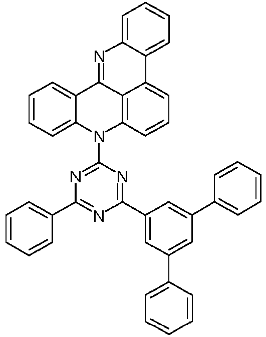 | 55 % |
| 22 | 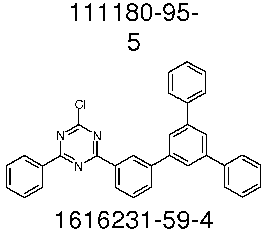 | 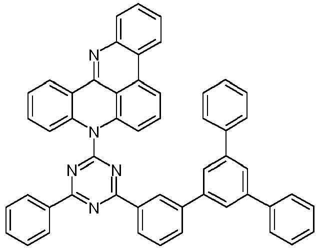 | 49 % |
| 23 | 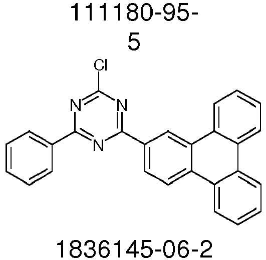 | 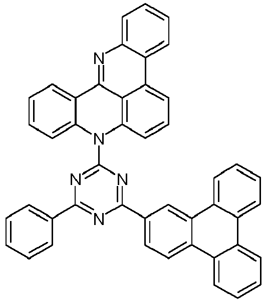 | 60 % |
| 24 | 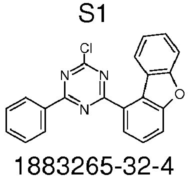 | 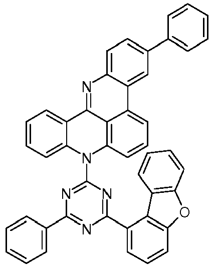 | 57 % |
| 25 | 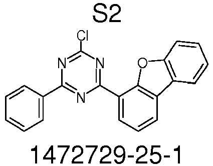 | 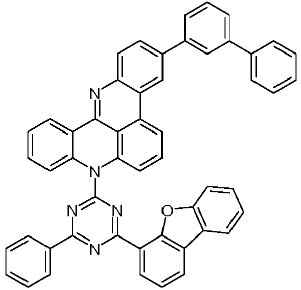 | 56 % |
| 26 | 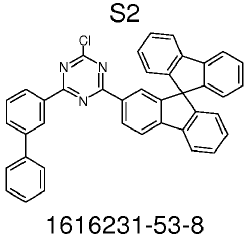 | 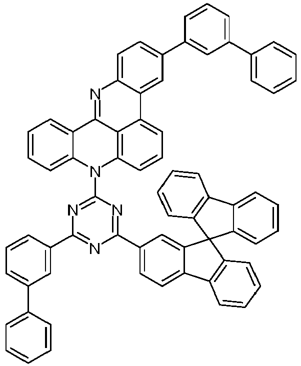 | 47 % |
| 27 | 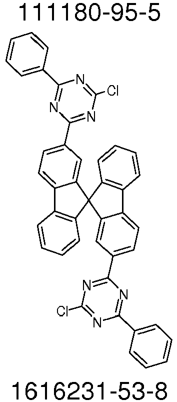 | 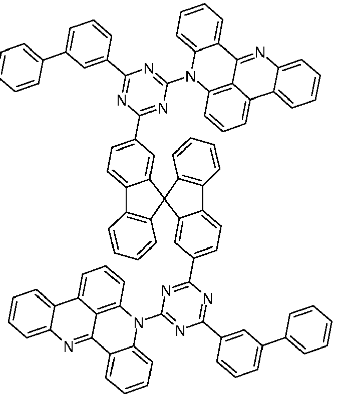 | 51 % |

### Beispiel 100:

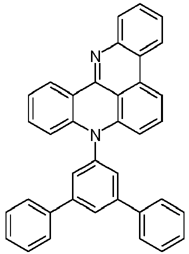

Ein Gemisch aus 26.8 g (100 mmol)) 8**H**-Chino[4,3,2-**kl**]acridin [111180-95-5], 35.6 g (100 mmol) 5'-Iod-1,1':3',1 "-terphenyl [87666-86-2], 27.6 g (200 mmol) Kaliumcarbonat, 28.4 g (200 mmol) Natriumsulfat, 1.3 g (20 mmol) Kupferpulver, 100 g Glaskugeln (3 mm Durchmesser) und 300 ml Nitrobenzol wird unter gutem Rühren 16 h unter Rückfluss erhitzt. Nach Erkalten versetzt man mit 1000 ml Methanol, saugt von den Feststoffen ab und wäscht diese dreimal mit je 300 ml Methanol. Die Feststoffe werden in 1000 ml Wasser heiß ausgerührt, nach Absaugen wäscht man zweimal mit je 200 ml heißem Wasser und dreimal mit je 200 ml Methanol nach und trocknet dann im Vakuum. Das so erhaltene Rohprodukt wird fünfmal mit o-Xylol (Vorlagemenge 250 ml) heißextrahiert und anschließend zweimal fraktioniert sublimiert (p ca. 10⁻⁵ mbar, T ca. 310 °C). Ausbeute: 28.3 g, (57 mmol), 57 %; Reinhei t: > 99.99 %ig nach HPLC.

Analog können folgende Verbindungen dargestellt werden:

| Bsp. | Edukte | Produkt | Ausbeute |
|---|---|---|---|
| 101 | 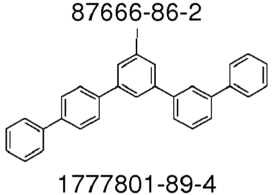 | 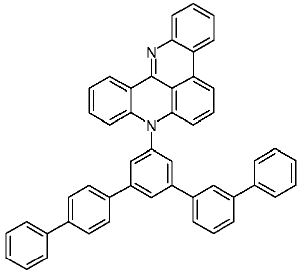 | 46 % |
| 102 | 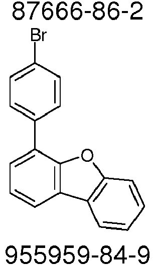 | 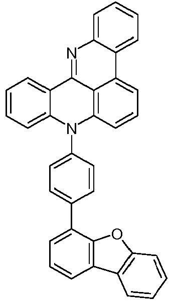 | 49 % |
| 103 | 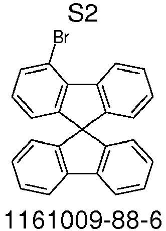 | 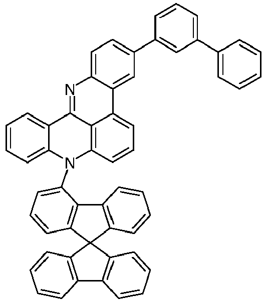 | 45 % |
| 104 | 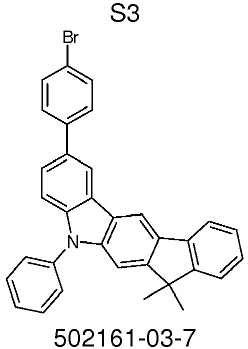 | 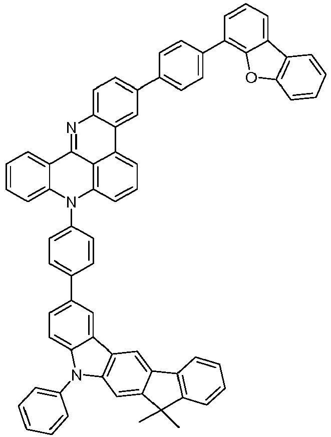 | 56 % |
| 105 | 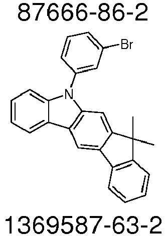 | 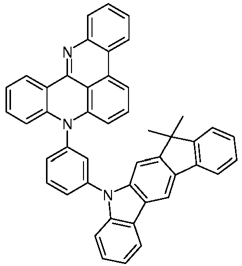 | 50 % |
| 106 | 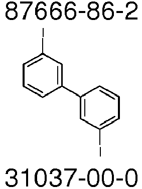 | 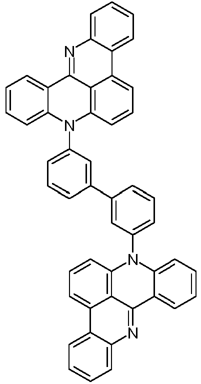 | 39 % |
| 107 | 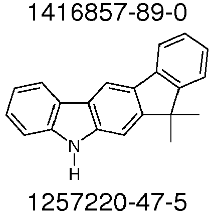 | 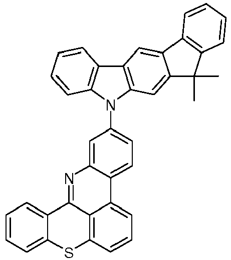 | 52 % |
| 108 | 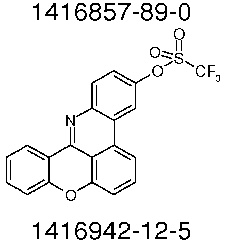 | 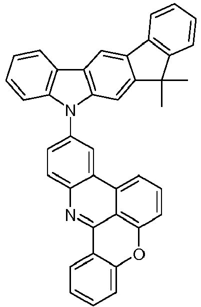 | 48 % |

### Herstellung der OLEDs

In den folgenden Beispielen E1 bis E22 wird die Verwendung verschiedener erfindungsgemäßer Materialien in OLEDs vorgestellt.

**Vorbehandlung für die Beispiele E1-E22:** Glasplättchen, die mit strukturiertem ITO (Indium Zinn Oxid) der Dicke 50 nm beschichtet sind, werden vor der Beschichtung mit einem Sauerstoffplasma, gefolgt von einem Argonplasma, behandelt. Diese mit Plasma behandelten Glasplättchen bilden die Substrate, auf welche die OLEDs aufgebracht werden.

Die OLEDs haben prinzipiell folgenden Schichtaufbau: Substrat / Lochinjektionsschicht (HIL) / Lochtransportschicht (HTL) / Elektronenblockierschicht (EBL) / Emissionsschicht (EML) / optionale Lochblockierschicht (HBL) / Elektronentransportschicht (ETL) / optionale Elektroneninjektionsschicht (EIL) und abschließend eine Kathode. Die Kathode wird durch eine 100 nm dicke Aluminiumschicht gebildet. Der genaue Aufbau der OLEDs ist Tabelle 1 zu entnehmen. Die zur Herstellung der OLEDs benötigten Materialien sind in Tabelle 2 gezeigt.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie IC5:IC3:TEG2 (55%:35%:10%) bedeutet hierbei, dass das Material IC5 in einem Volumenanteil von 55%, IC3 in einem Anteil von 35% und TEG2 in einem Anteil von 10% in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren, die Stromeffizienz (gemessen in cd/A), die Leistungseffizienz (gemessen in lm/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik sowie die Lebensdauer bestimmt. Die Elektrolumineszenzspektren werden bei einer Leuchtdichte von 1000 cd/m² bestimmt und daraus die CIE 1931 x und y Farbkoordinaten berechnet.

### Verwendung von erfindungsgemäßen Mischungen in der Emissionsschicht phosphoreszenter OLEDs

Die erfindungsgemäßen Materialien können in der Emissionsschicht in phosphoreszierenden roten OLEDs eingesetzt werden. Das erfindungsgemäße Material EG1 ist in Beispiel E1 als Matrixmaterial in Kombination mit dem phosphoreszenten Emitter TEG5 eingesetzt. Bei einer Leuchtdichte von 1000 cd/m² hat die OLED aus E1 eine Spannung von 3.8 V, eine EQE von 21% und Farbkoordinaten von CIEx=0.67 und CIEy=0.33.

Auch in den Beispielen E2 bis E16 emittiert die OLED Licht mit den Farbkoordinaten CIEx = 0.67 und CIEy = 0.33. Dies zeigt, dass sich die erfindungsgemäßen Verbindungen EG1-EG16 zum Einsatz als Matrixmaterial in OLEDs eignen.

### Verwendung von erfindungsgemäßen Mischungen in der Elektronentransportschicht phosphoreszenter OLEDs

Die erfindungsgemäßen Materialien können auch in der Elektronentransportschicht in OLEDs eingesetzt werden. In den Beispielen E17 bis E22 werden die erfindungsgemäßen Materialien EG17 bis EG22 in der Elektronentransportschicht eingesetzt. In den Beispielen E17 bis E22 emittiert die OLED Licht mit den Farbkoordinaten CIEx = 0.67 und CIEy = 0.33. Dies zeigt, dass sich die erfindungsgemäßen Verbindungen EG17 bis EG22 zum Einsatz als Elektronentransportmaterial in OLEDs eignen.

**Tabelle 1: Aufbau der OLEDs**

| Bsp | HIL Dicke | HTL Dicke | EBL Dicke | EML Dicke | HBL Dicke | ETL Dicke | EIL Dicke |
|---|---|---|---|---|---|---|---|
| E1 | HATCN | SpMA1 | SpMA3 | EG1:TER5 | | ST2:LiQ (50%:50%) | |
| | 5nm | 125nm | 10nm | (95%:5%) 40nm | | 35nm | |
| E2 | HATCN | SpMA1 | SpMA3 | EG2:TER5 | | ST2:LiQ (50%:50%) | |
| | 5nm | 125nm | 10nm | (95%:5%) 40nm | | 35nm | |
| E3 | HATCN | SpMA1 | SpMA3 | EG1:EG3:TER5 | | ST2:LiQ (50%:50%) | |
| | 5nm | 125nm | 10nm | (85%10%:5%) 40nm | | 35nm | |
| E4 | HATCN | SpMA1 | SpMA3 | EG1:EG4:TER5 | | ST2:LiQ (50%:50%) | |
| | 5nm | 125nm | 10nm | (85%10%:5%) 40nm | | 35nm | |
| E5 | HATCN | SpMA1 | SpMA3 | EG5:TER5 | | ST2:LiQ (50%:50%) | |
| | 5nm | 125nm | 10nm | (95%:5%) 40nm | | 35nm | |
| E6 | HATCN | SpMA1 | SpMA3 | EG6:TER5 | | ST2:LiQ (50%:50%) | |
| | 5nm | 125nm | 10nm | (95%:5%) 40nm | | 35nm | |
| E7 | HATCN | SpMA1 | SpMA3 | EG7:TER5 | | ST2:LiQ (50%:50%) | |
| | 5nm | 125nm | 10nm | (95%:5%) 40nm | | 35nm | |
| E8 | HATCN | SpMA1 | SpMA3 | EG8:TER5 | | ST2:LiQ (50%:50%) | |
| | 5nm | 125nm | 10nm | (95%:5%) 40nm | | 35nm | |
| E9 | HATCN | SpMA1 | SpMA3 | EG9:TER5 | | ST2:LiQ (50%:50%) | |
| | 5nm | 125nm | 10nm | (95%:5%) 40nm | | 35nm | |
| E10 | HATCN | SpMA1 | SpMA3 | EG10:TER5 | | ST2:LiQ (50%:50%) | |
| | 5nm | 125nm | 10nm | (95%:5%) 40nm | | 35nm | |
| E11 | HATCN | SpMA1 | SpMA3 | EG11:TER5 | | ST2:LiQ (50%:50%) | |
| | 5nm | 125nm | 10nm | (95%:5%) 40nm | | 35nm | |
| E12 | HATCN | SpMA1 | SpMA3 | EG12:TER5 | | ST2:LiQ (50%:50%) | |
| | 5nm | 125nm | 10nm | (95%:5%) 40nm | | 35nm | |
| E13 | HATCN | SpMA1 | SpMA3 | EG1:EG13:TER5 | | ST2:LiQ (50%:50%) | |
| | 5nm | 125nm | 10nm | (85%10%:5%) 40nm | | 35nm | |
| E14 | HATCN | SpMA1 | SpMA3 | EG1:EG14:TER5 | | ST2:LiQ (50%:50%) | |
| | 5nm | 125nm | 10nm | (85%10%:5%) 40nm | | 35nm | |
| E15 | HATCN | SpMA1 | SpMA3 | EG1:EG15:TER5 | | ST2:LiQ (50%:50%) | |
| | 5nm | 125nm | 10nm | (85%10%:5%) 40nm | | 35nm | |
| E16 | HATCN | SpMA1 | SpMA3 | EG1:EG16:TER5 | | ST2:LiQ (50%:50%) | |
| | 5nm | 125nm | 10nm | (85%10%:5%) 40nm | | 35nm | |
| E17 | HATCN | SpMA1 | SpMA3 | EG1:TER5 | ST2 | ST2:EG17 (50%:50%) | LiQ |
| | 5nm | 125nm | 10nm | (95%:5%) 40nm | 5nm | 30nm | 3nm |
| E18 | HATCN | SpMA1 | SpMA3 | EG1:TER5 | ST2 | ST2:EG18 (50%:50%) | LiQ |
| | 5nm | 125nm | 10nm | (95%:5%) 40nm | 5nm | 30nm | 3nm |
| E19 | HATCN | SpMA1 | SpMA3 | EG1:TER5 | ST2 | ST2:EG19 (50%:50%) | LiQ |
| | 5nm | 125nm | 10nm | (95%:5%) 40nm | 5nm | 30nm | 3nm |
| E20 | HATCN | SpMA1 | SpMA3 | EG1:TER5 | ST2 | ST2:EG20 (50%:50%) | LiQ |
| | 5nm | 125nm | 10nm | (95%:5%) 40nm | 5nm | 30nm | 3nm |
| E21 | HATCN | SpMA1 | SpMA3 | EG1:TER5 | ST2 | ST2:EG21 (50%:50%) | LiQ |
| | 5nm | 125nm | 10nm | (95%:5%) 40nm | 5nm | 30nm | 3nm |
| E22 | HATCN | SpMA1 | SpMA3 | EG1:TER5 | ST2 | ST2:EG22 (50%:50%) | LiQ |
| | 5nm | 125nm | 10nm | (95%:5%) 40nm | 5nm | 30nm | 3nm |

**Tabelle 2: Strukturformeln der Materialien für die OLEDs**

| | |
|---|---|
| 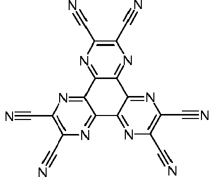 | 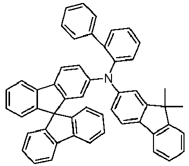 |
| HATCN | SpMA1 |
| 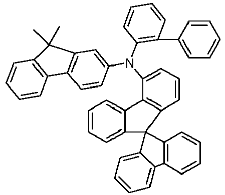 | 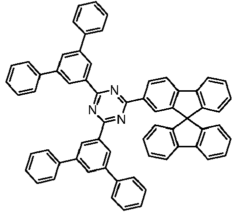 |
| SpMA3 | ST2 |
| 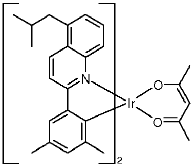 | 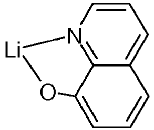 |
| TER5 | LiQ |
| 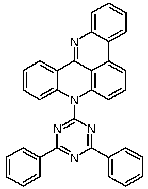 | 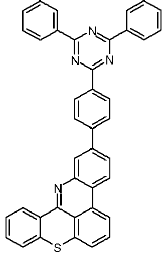 |
| EG1 | EG2 |
| 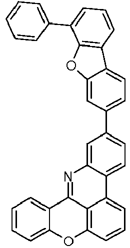 | 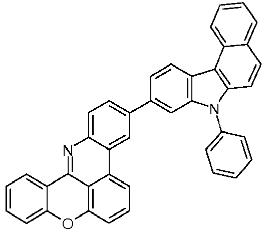 |
| EG3 | EG4 |
| 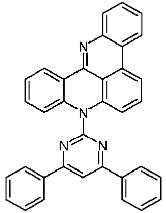 | 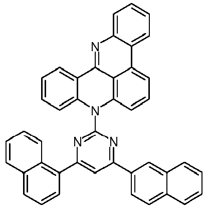 |
| EG5 | EG6 |
| 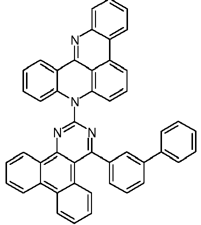 | 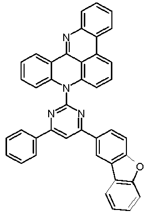 |
| EG7 | EG8 |
| 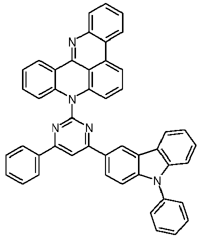 | 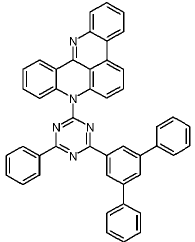 |
| EG9 | EG10 |
| 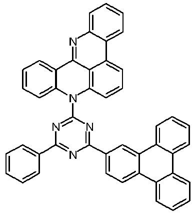 | 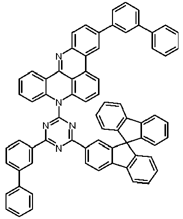 |
| EG11 | EG12 |
| 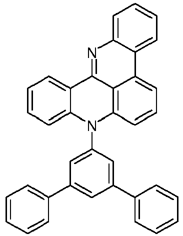 | 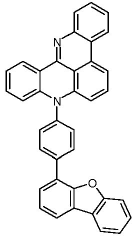 |
| EG13 | EG14 |
| 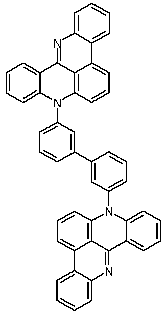 | 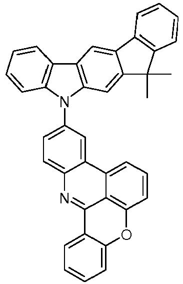 |
| EG15 | EG16 |
| 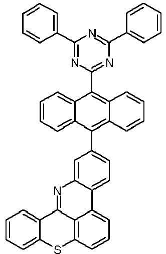 | 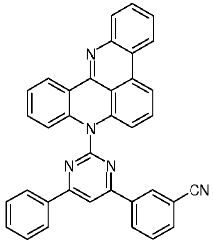 |
| EG17 | EG18 |
| 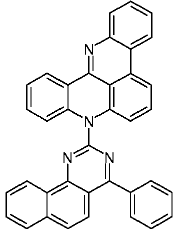 | 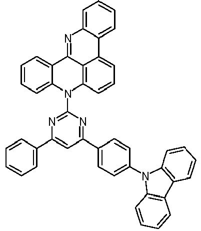 |
| EG19 | EG20 |
| 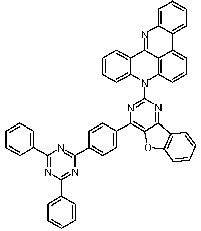 | 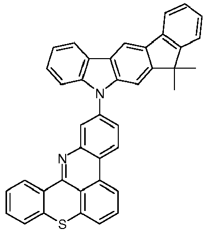 |
| EG21 | EG22 |

## Patentansprüche

1. Verbindung gemäß Formel (1), 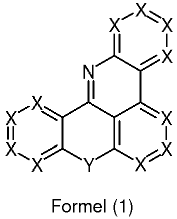 wobei für die verwendeten Symbole gilt:
X ist bei jedem Auftreten gleich oder verschieden CR oder N;
Y ist NR', BR', O oder S;
R ist bei jedem Auftreten gleich oder verschieden H, D, F, Cl, Br, I, NAr₂, N(R¹)₂, CN, NO₂, OR¹, SR¹, COOR¹, C(=O)N(R¹)₂, Si(R¹)₃, B(OR¹)₂, C(=O)R¹, P(=O)(R¹)₂, S(=O)R¹, S(=O)₂R¹, OSO₂R¹, eine geradkettige Alkylgruppe mit 1 bis 20 C-Atomen oder eine Alkenyl- oder Alkinylgruppe mit 2 bis 20 C-Atomen oder eine verzweigte oder cyclische Alkylgruppe mit 3 bis 20 C-Atomen, wobei die Alkyl-, Alkenyl- oder Alkinylgruppe jeweils mit einem oder mehreren Resten R¹ substituiert sein kann und wobei eine oder mehrere nicht benachbarte CH₂-Gruppen durch Si(R¹)₂, C=O, NR¹, O, S oder CONR¹ ersetzt sein können, oder ein aromatisches oder heteroaromatisches Ringsystem mit 5 bis 60 aromatischen Ringatomen, das jeweils durch einen oder mehrere Reste R¹ substituiert sein kann; dabei können zwei Reste R auch miteinander ein aliphatisches oder heteroaliphatisches Ringsystem bilden;
R' ist ein aromatisches oder heteroaromatisches Ringsystem mit 5 bis 40 aromatischen Ringatomen, das durch einen oder mehrere Reste R¹ substituiert sein kann;
Ar ist bei jedem Auftreten gleich oder verschieden ein aromatisches oder heteroaromatisches Ringsystem mit 5 bis 40 aromatischen Ringatomen, das durch einen oder mehrere Reste R¹ substituiert sein kann;
R¹ ist bei jedem Auftreten gleich oder verschieden H, D, F, Cl, Br, I, N(R²)₂, CN, NO₂, OR², SR², Si(R²)₃, B(OR²)₂, C(=O)R², P(=O)(R²)₂, S(=O)R², S(=O)₂R², OSO₂R², eine geradkettige Alkylgruppe mit 1 bis 20 C-Atomen oder eine Alkenyl- oder Alkinylgruppe mit 2 bis 20 C-Atomen oder eine verzweigte oder cyclische Alkylgruppe mit 3 bis 20 C-Atomen, wobei die Alkyl-, Alkenyl- oder Alkinylgruppe jeweils mit einem oder mehreren Resten R² substituiert sein kann und wobei eine oder mehrere nicht benachbarte CH₂-Gruppen durch Si(R²)₂, C=O, NR², O, S oder CONR² ersetzt sein können, oder ein aromatisches oder heteroaromatisches Ringsystem mit 5 bis 40 aromatischen Ringatomen, das jeweils durch einen oder mehrere Reste R² substituiert sein kann; dabei können zwei oder mehrere Reste R¹ miteinander ein Ringsystem bilden;
R² ist bei jedem Auftreten gleich oder verschieden H, D, F oder ein aliphatischer, aromatischer oder heteroaromatischer organischer Rest mit 1 bis 20 C-Atomen, in dem auch ein oder mehrere H-Atome durch F ersetzt sein können;
mit der Maßgabe, dass die Verbindung der Formel (1) mindestens einen Substituenten R' enthält und/oder dass die Verbindung der Formel (1) mindestens einen Substituenten R enthält, der ausgewählt ist aus der Gruppe bestehend aus NAr₂ oder einem aromatischen oder heteroaromatischen Ringsystem mit 5 bis 60 aromatischen Ringatomen, welches durch einen oder mehrere Reste R¹ substituiert sein kann.

2. Verbindung nach Anspruch 1 gemäß Formel (1') oder (1"), 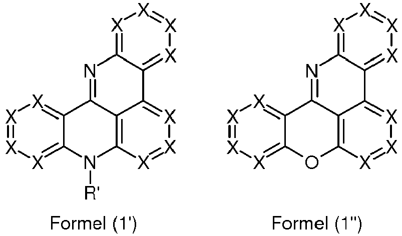 wobei die Symbole die in Anspruch 1 genannten Bedeutungen aufweisen und die Verbindung der Formel (1") mindestens einen Rest R aufweist, der ausgewählt ist aus der Gruppe bestehend aus NAr₂ und einem aromatischen oder heteroaromatischen Ringsystem mit 5 bis 60 aromatischen Ringatomen, welches durch einen oder mehrere Reste R¹ substituiert sein kann.

3. Verbindung nach Anspruch 1 oder 2, **dadurch gekennzeichnet, dass** maximal ein Symbol X pro Ring für N steht und die verbleibenden Symbole X für CR stehen.

4. Verbindung nach einem oder mehreren der Ansprüche 1 bis 3 gemäß einer der Formeln (2a) bis (2h), 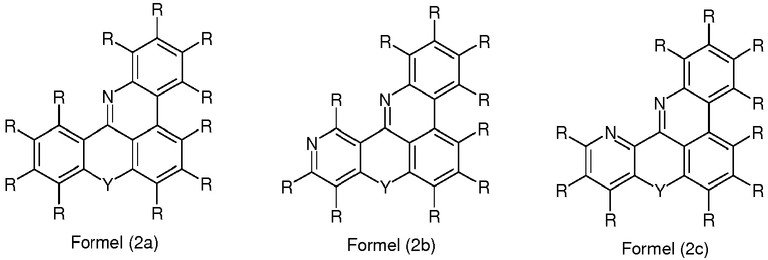 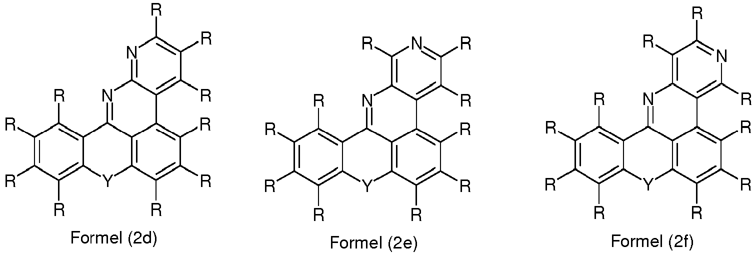 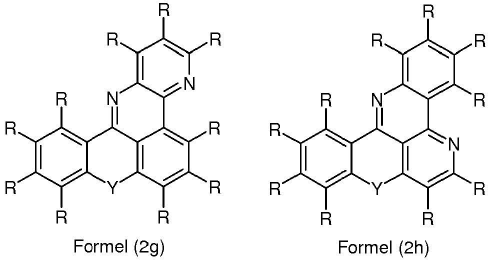 wobei die Symbole die in Anspruch 1 genannten Bedeutungen aufweisen.

5. Verbindung nach einem oder mehreren der Ansprüche 1 bis 4 gemäß einer der Formeln (2a') oder (2a"), 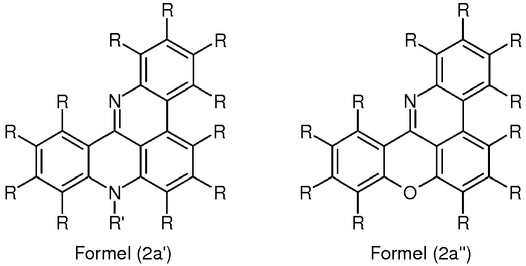 wobei die Symbole die in Anspruch 1 genannten Bedeutungen aufweisen und mindestens eine Gruppe R in Formel (2a") ausgewählt ist aus der Gruppe bestehend aus NAr₂ und einem aromatischen oder heteroaromatischen Ringsystem mit 5 bis 60 aromatischen Ringatomen, welches durch einen oder mehrere Reste R¹ substituiert sein kann.

6. Verbindung nach einem oder mehreren der Ansprüche 1 bis 5 gemäß Formel (3), 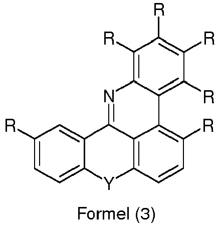 wobei die Symbole die in Anspruch 1 genannten Bedeutungen aufweisen.

7. Verbindung nach einem oder mehreren der Ansprüche 1 bis 6 gemäß einer der Formeln (3') oder (3"), 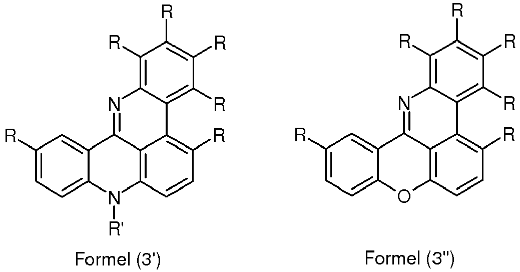 wobei die Symbole die in Anspruch 1 genannten Bedeutungen aufweisen und mindestens eine Gruppe R in Formel (3") ausgewählt ist aus der Gruppe bestehend aus NAr₂ und einem aromatischen oder heteroaromatischen Ringsystem mit 5 bis 60 aromatischen Ringatomen, welches durch einen oder mehrere Reste R¹ substituiert sein kann.

8. Verbindung nach einem oder mehreren der Ansprüche 1 bis 7 gemäß Formel (4), 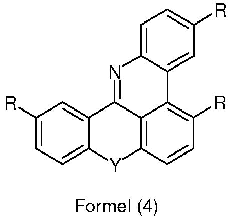 wobei die Symbole die in Anspruch 1 genannten Bedeutungen aufweisen.

9. Verbindung nach einem oder mehreren der Ansprüche 1 bis 8 gemäß einer der Formeln (4') oder (4"), 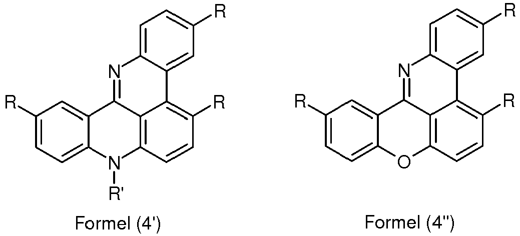 wobei die Symbole die in Anspruch 1 genannten Bedeutungen aufweisen und mindestens eine Gruppe R in Formel (4") ausgewählt ist aus der Gruppe bestehend aus NAr₂ und einem aromatischen oder heteroaromatischen Ringsystem mit 5 bis 60 aromatischen Ringatomen, welches durch einen oder mehrere Reste R¹ substituiert sein kann.

10. Verbindung nach einem oder mehreren der Ansprüche 1 bis 9, **dadurch gekennzeichnet, dass** R, wenn dieses für ein aromatisches oder heteroaromatisches Ringsystem steht, R' und/oder Ar ausgewählt sind aus der Gruppe bestehend aus Phenyl, Biphenyl, Terphenyl, Quaterphenyl, Fluoren, Spirobifluoren, Naphthalin, Indol, Benzofuran, Benzothiophen, Carbazol, Dibenzofuran, Dibenzothiophen, Indenocarbazol, Indolocarbazol, Pyridin, Pyrimidin, Pyrazin, Pyridazin, Triazin, Phenanthren, Triphenylen oder einer Kombination aus zwei oder drei dieser Gruppen, welche jeweils mit einem oder mehreren Resten R¹ substituiert sein können.

11. Verfahren zur Herstellung einer Verbindung nach einem oder mehreren der Ansprüche 1 bis 10, umfassend die Reaktionsschritte:
a) Bereitstellung des Grundgerüsts mit einer reaktiven Abgangsgruppe; und
b) Kupplung eines aromatischen bzw. heteroaromatischen Ringsystems bzw. einer Verbindung H-NAr₂ mit dem Grundgerüst unter Abspaltung der Abgangsgruppe.

12. Formulierung enthaltend mindestens eine Verbindung nach einem oder mehreren der Ansprüche 1 bis 10 und mindestens ein Lösemittel und/oder mindestens eine weitere organische oder anorganische Verbindung sein.

13. Verwendung einer Verbindung nach einem oder mehreren der Ansprüche 1 bis 10 in einer elektronischen Vorrichtung, insbesondere in einer organischen Elektrolumineszenzvorrichtung.

14. Elektronische Vorrichtung enthaltend mindestens eine Verbindung nach einem oder mehreren der Ansprüche 1 bis 10.

15. Elektronische Vorrichtung nach Anspruch 14, wobei es sich um eine organische Elektrolumineszenzvorrichtung handelt, **dadurch gekennzeichnet, dass** die Verbindung nach einem oder mehreren der Ansprüche 1 bis 10 als Matrixmaterial und/oder in einer Lochblockierschicht und/oder in einer Elektronentransportschicht eingesetzt wird.

## Claims

1. Compound of formula (1) 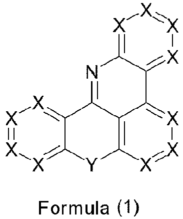 where the symbols used are as follows:
X is the same or different at each instance and is CR or N;
Y is NR', BR', 0 or S;
R is the same or different at each instance and is H, D, F, Cl, Br, I, NAr₂, N(R¹)₂, CN, NO₂, OR¹, SR¹, COOR¹, C(=O)N(R¹)₂, Si(R¹)₃, B(OR¹)₂, C(=O)R¹, P(=O)(R¹)₂, S(=O)R¹, S(=O)₂R¹, OSO₂R¹, a straight-chain alkyl group having 1 to 20 carbon atoms or an alkenyl or alkynyl group having 2 to 20 carbon atoms or a branched or cyclic alkyl group having 3 to 20 carbon atoms, where the alkyl, alkenyl or alkynyl group may in each case be substituted by one or more R¹ radicals and where one or more nonadjacent CH₂ groups may be replaced by Si(R¹)₂, C=0, NR¹, 0, S or CONR¹, or an aromatic or heteroaromatic ring system which has 5 to 60 aromatic ring atoms and may be substituted in each case by one or more R¹ radicals; at the same time, two R radicals together may also form an aliphatic or heteroaliphatic ring system;
R' is an aromatic or heteroaromatic ring system which has 5 to 40 aromatic ring atoms and may be substituted by one or more R¹ radicals;
Ar is the same or different at each instance and is an aromatic or heteroaromatic ring system which has 5 to 40 aromatic ring atoms and may be substituted by one or more R¹ radicals;
R¹ is the same or different at each instance and is H, D, F, Cl, Br, I, N(R²)₂, CN, NO₂, OR², SR², Si(R²)₃, B(OR²)₂, C(=O)R², P(=O)(R²)₂, S(=O)R², S(=O)₂R², OSO₂R², a straight-chain alkyl group having 1 to 20 carbon atoms or an alkenyl or alkynyl group having 2 to 20 carbon atoms or a branched or cyclic alkyl group having 3 to 20 carbon atoms, where the alkyl, alkenyl or alkynyl group may in each case be substituted by one or more R² radicals and where one or more nonadjacent CH₂ groups may be replaced by Si(R²)₂, C=O, NR², O, S or CONR², or an aromatic or heteroaromatic ring system which has 5 to 40 aromatic ring atoms and may be substituted in each case by one or more R² radicals; at the same time, two or more R¹ radicals together may form a ring system;
R² is the same or different at each instance and is H, D, F or an aliphatic, aromatic or heteroaromatic organic radical having 1 to 20 carbon atoms, in which one or more hydrogen atoms may also be replaced by F;
with the proviso that the compound of the formula (1) contains at least one substituent R' and/or that the compound of the formula (1) contains at least one substituent R selected from the group consisting of NAr₂ and an aromatic or heteroaromatic ring system which has 5 to 60 aromatic ring atoms and may be substituted by one or more R¹ radicals.

2. Compound according to Claim 1 of formula (1') or (1") 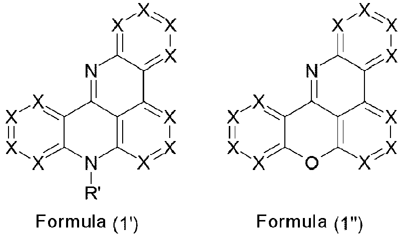 where the symbols have the definitions given in Claim 1 and the compound of the formula (1") has at least one R radical selected from the group consisting of NAr₂ and an aromatic or heteroaromatic ring system which has 5 to 60 aromatic ring atoms and may be substituted by one or more R¹ radicals.

3. Compound according to Claim 1 or 2, **characterized in that** not more than one symbol X per ring is N and the remaining symbols X are CR.

4. Compound according to one or more of Claims 1 to 3 of one of the formulae (2a) to (2h) 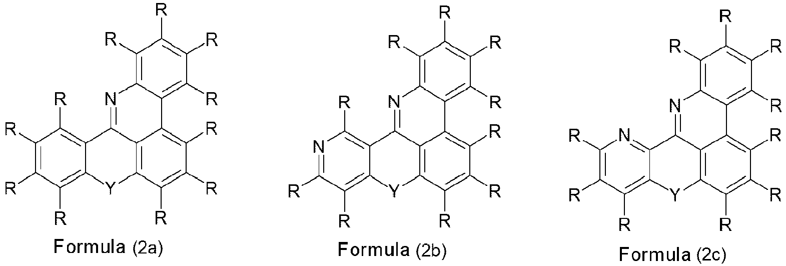 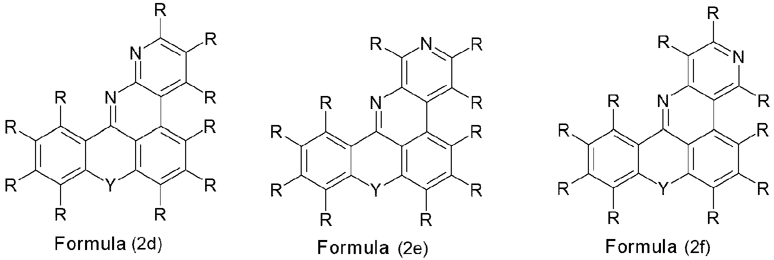 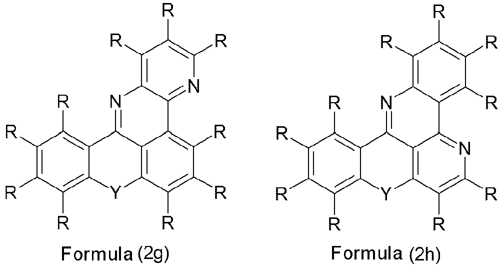 where the symbols have the definitions given in Claim 1.

5. Compound according to one or more of Claims 1 to 4 of one of the formulae (2a') and (2a'') 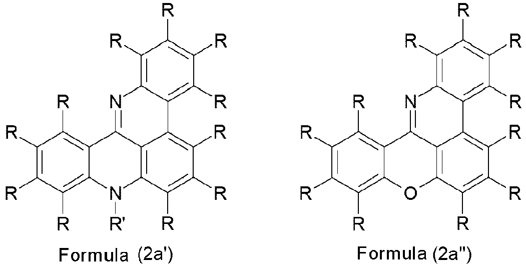 where the symbols have the definitions given in Claim 1 and at least one R group in formula (2a") is selected from the group consisting of NAr₂ and an aromatic or heteroaromatic ring system which has 5 to 60 aromatic ring atoms and may be substituted by one or more R¹ radicals.

6. Compound according to one or more of Claims 1 to 5 of formula (3) 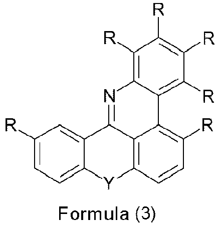 where the symbols have the definitions given in Claim 1.

7. Compound according to one or more of Claims 1 to 6 of one of the formulae (3') and (3") 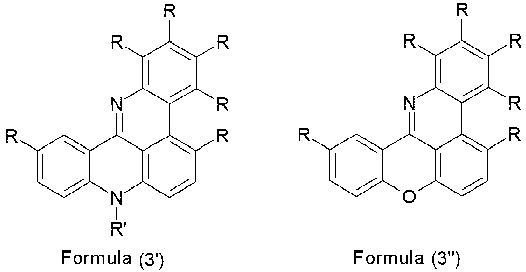 where the symbols have the definitions given in Claim 1 and at least one R group in formula (3") is selected from the group consisting of NAr₂ and an aromatic or heteroaromatic ring system which has 5 to 60 aromatic ring atoms and may be substituted by one or more R¹ radicals.

8. Compound according to one or more of Claims 1 to 7 of formula (4) 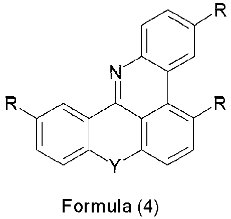 where the symbols have the definitions given in Claim 1.

9. Compound according to one or more of Claims 1 to 8 of one of the formulae (4') and (4") 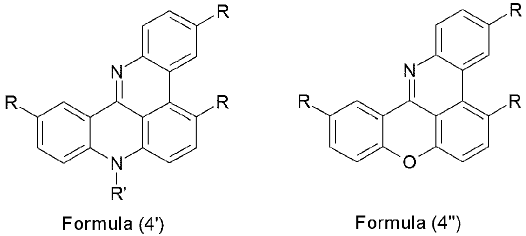 where the symbols have the definitions given in Claim 1 and at least one R group in formula (4") is selected from the group consisting of NAr₂ and an aromatic or heteroaromatic ring system which has 5 to 60 aromatic ring atoms and may be substituted by one or more R¹ radicals.

10. Compound according to one or more of Claims 1 to 9, **characterized in that** R, when it is an aromatic or heteroaromatic ring system, R' and/or Ar are selected from the group consisting of phenyl, biphenyl, terphenyl, quaterphenyl, fluorene, spirobifluorene, naphthalene, indole, benzofuran, benzothiophene, carbazole, dibenzofuran, dibenzothiophene, indenocarbazole, indolocarbazole, pyridine, pyrimidine, pyrazine, pyridazine, triazine, phenanthrene, triphenylene or a combination of two or three of these groups, each of which may be substituted by one or more R¹ radicals.

11. Process for preparing a compound according to one or more of Claims 1 to 10, comprising the reaction steps of:
a) providing the base skeleton having a reactive leaving group; and
b) coupling an aromatic or heteroaromatic ring system or a compound H-NAr₂ to the base skeleton with detachment of the leaving group.

12. Formulation comprising at least one compound according to one or more of Claims 1 to 10 and at least one solvent and/or at least one further organic or inorganic compound.

13. Use of a compound according to one or more of Claims 1 to 10 in an electronic device, especially in an organic electroluminescent device.

14. Electronic device comprising at least one compound according to one or more of Claims 1 to 10.

15. Electronic device according to Claim 14 which is an organic electroluminescent device, **characterized in that** the compound according to one or more of Claims 1 to 10 is used as matrix material and/or in a hole blocker layer and/or in an electron transport layer.

## Revendications

1. Composé de formule (1) 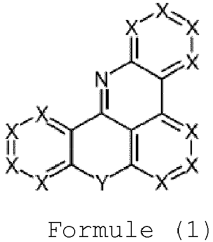 dans laquelle les symboles utilisés ont les significations suivantes :
X à chaque occurrence, est identique ou différent et représente CR ou N ;
Y représente NR', BR', O ou S ;
R à chaque occurrence, est identique ou différent et représente H, D, F, Cl, Br, I, NAr₂, N(R¹)₂, CN, NO₂, OR¹, SR¹, COOR¹, C(=O)N(R¹)₂, Si(R¹)₃, B(OR¹)₂, C(=O)R¹, P(=O)(R¹)₂, S(=O)R¹, S(=O)₂R¹, OSO₂R¹, un groupe alkyle à chaîne droite ayant 1 à 20 atomes de carbone ou un groupe alcényle ou alcynyle ayant 2 à 20 atomes de carbone ou un groupe alkyle ramifié ou cyclique ayant 3 à 20 atomes de carbone, chaque groupe alkyle, alcényle ou alcynyle pouvant être substitué par un ou plusieurs radicaux R¹, et un ou plusieurs groupes CH₂ non-voisins peuvent être remplacés par Si(R¹)₂, C=O, NR¹, O, S ou CONR¹, ou un système cyclique aromatique ou hétéroaromatique ayant 5 à 60 atomes nucléaires aromatiques, dont chacun peut être substitué par un ou plusieurs radicaux R¹ ; deux radicaux R pouvant alors former aussi ensemble un système cyclique aliphatique ou hétéroaliphatique ;
R' représente un système cyclique aromatique ou hétéroaromatique ayant 5 à 40 atomes nucléaires aromatiques, qui peut être substitué par un ou plusieurs radicaux R¹ ;
Ar à chaque occurrence, est identique ou différent et représente un système cyclique aromatique ou hétéroaromatique ayant 5 à 40 atomes nucléaires aromatiques, qui peut être substitué par un ou plusieurs radicaux R¹ ;
R¹ à chaque occurrence, est identique ou différent et représente H, D, F, Cl, Br, I, N(R²)₂, CN, NO₂, OR², SR², Si(R²)₃, B(OR²)₂, C(=O)R², P(=O)(R²)₂, S(=O)R², S(=O)₂R², OSO₂R², un groupe alkyle à chaîne droite ayant 1 à 20 atomes de carbone ou un groupe alcényle ou alcynyle ayant 2 à 20 atomes de carbone ou un groupe alkyle ramifié ou cyclique ayant 3 à 20 atomes de carbone, chaque groupe alkyle, alcényle ou alcynyle pouvant être substitué par un ou plusieurs radicaux R², et un ou plusieurs groupes CH₂ non-voisins pouvant être remplacés par Si(R²)₂, C=O, NR², O, S ou CONR², ou un système cyclique aromatique ou hétéroaromatique ayant 5 à 40 atomes nucléaires aromatiques, dont chacun peut être substitué par un ou plusieurs radicaux R² ; deux ou plusieurs radicaux R¹ peuvent alors former ensemble un système cyclique ;
R² à chaque occurrence, est identique ou différent et représente H, D, F ou un radical organique aliphatique, aromatique ou hétéroaromatique ayant 1 à 20 atomes de carbone, dans lequel de même un ou plusieurs atomes H peuvent être remplacés par F ;
à la condition que le composé de formule (1) contienne au moins un substituant R', et que le composé de formule (1) contienne au moins un substituant R, qui est choisi dans le groupe consistant en NAr₂ ou un système cyclique aromatique ou hétéroaromatique ayant 5 à 60 atomes nucléaires aromatiques, qui peut être substitué par un ou plusieurs radicaux R¹.

2. Composé selon la revendication 1 selon la formule (1') ou (1"), 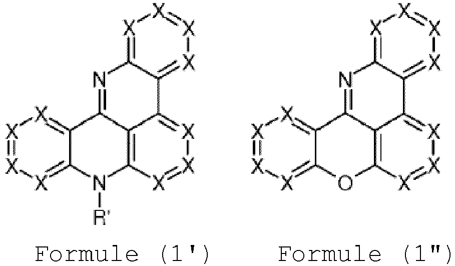 dans laquelle les symboles ont les significations données dans la revendication 1, et le composé de formule (1") comprend au moins un radical R, qui est choisi dans le groupe consistant en NAr₂ et un système cyclique aromatique ou hétéroaromatique ayant 5 à 60 atomes nucléaires aromatiques, qui peut être substitué par un ou plusieurs radicaux R¹.

3. Composé selon la revendication 1 ou 2, **caractérisé en ce qu'**au maximum un symbole X par cycle représente N, les autres symboles X représentant CR.

4. Composé selon l'une ou plusieurs des revendications 1 à 3 selon l'une des formules (2a) à (2h), 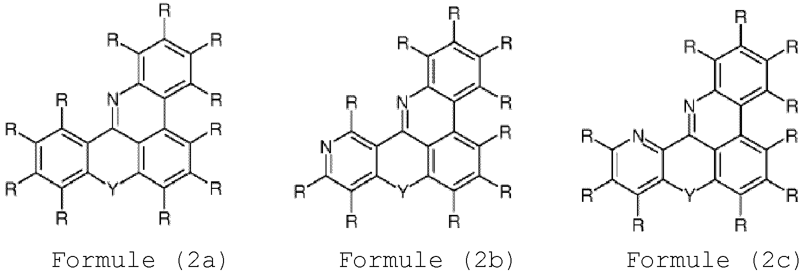 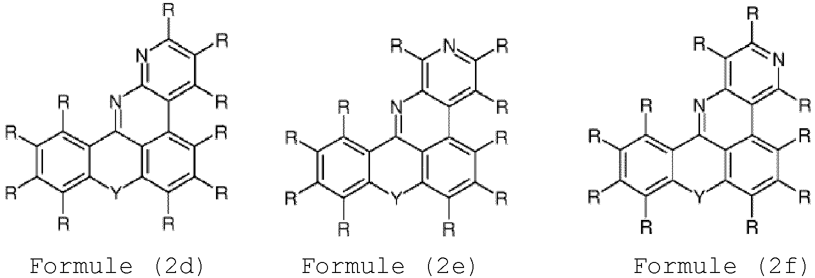 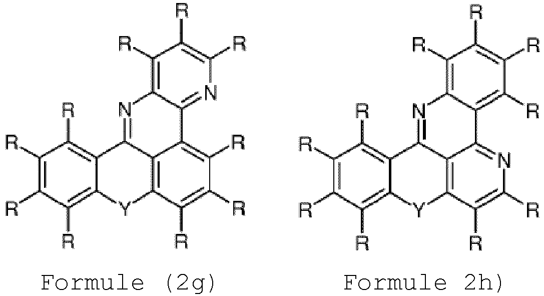 dans lesquelles les symboles ont les significations données dans la revendication 1.

5. Composé selon l'une ou plusieurs des revendications 1 à 4 selon l'une des formules (2a') ou (2a"), 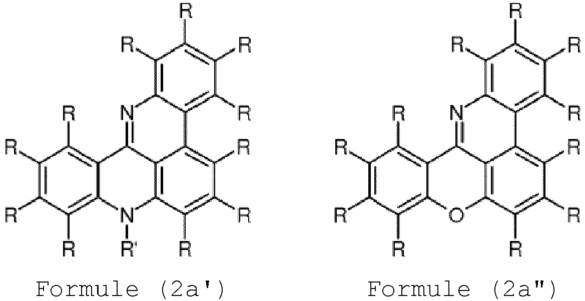 dans laquelle les symboles ont les significations données dans la revendication 1, et au moins un groupe R, dans la formule (2a"), est choisi dans le groupe consistant en NAr₂ et un système cyclique aromatique ou hétéroaromatique ayant 5 à 60 atomes nucléaires aromatiques, qui peut être substitué par un ou plusieurs radicaux R¹.

6. Composé selon l'une ou plusieurs des revendications 1 à 5 selon la formule (3), 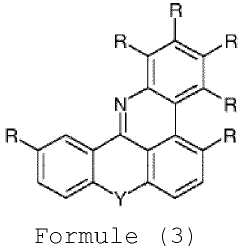 dans laquelle les symboles ont les significations données dans la revendication 1.

7. Composé selon l'une des revendications 1 à 6 selon l'une des formules (3') ou (3"), 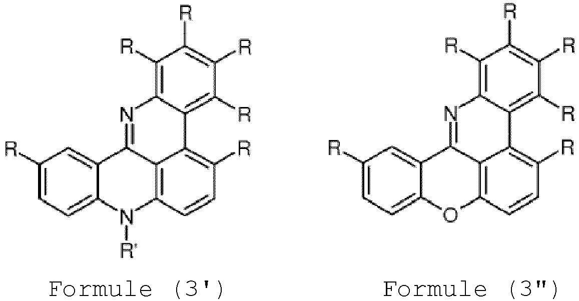 dans laquelle les symboles ont les significations données dans la revendication 1, et au moins un groupe R, dans la formule (3"), est choisi dans le groupe consistant en NAr₂ et un système cyclique aromatique ou hétéroaromatique ayant 5 à 60 atomes nucléaires aromatiques, qui peut être substitué par un ou plusieurs radicaux R¹.

8. Composé selon l'une ou plusieurs des revendications 1 à 7 selon la formule (4), 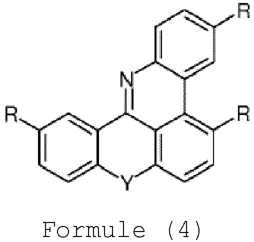 dans laquelle les symboles ont les significations données dans la revendication 1.

9. Composé selon l'une ou plusieurs des revendications 1 à 8 selon l'une des formules (4') ou (4"), 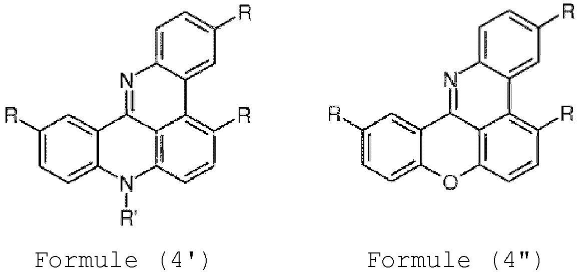 dans laquelle les symboles ont les significations données dans la revendication 1, et au moins un groupe R, dans la formule (4"), est choisi dans le groupe consistant en NAr₂ et un système cyclique aromatique ou hétéroaromatique ayant 5 à 60 atomes nucléaires aromatiques, qui peut être substitué par un ou plusieurs radicaux R¹.

10. Composé selon l'une ou plusieurs des revendications 1 à 9, **caractérisé en ce que** R, quand ce dernier représente un système cyclique aromatique ou hétéroaromatique, R' et/ou Ar sont choisis dans le groupe consistant en un phényle, un biphényle, un terphényle, un quaterphényle, un fluorène, un spirobifluorène, un naphtalène, un indol, un benzofuranne, un benzothiophène, un carbazole, un dibenzofuranne, un dibenzothiophène, un indénocarbazole, un indolocarbazole, une pyridine, une pyrimidine, une pyrazine, une pyridazine, une triazine, un phénanthrène, un triphénylène ou une combinaison de deux ou trois de ces groupes, dont chacun peut être substitué par un ou plusieurs radicaux R¹.

11. Procédé de fabrication d'un composé selon l'une ou plusieurs des revendications 1 à 10, comprenant les étapes de réaction :
a) fourniture d'un squelette de base comportant un groupe partant réactif ; et
b) couplage d'un système cyclique aromatique ou hétéroaromatique ou d'un composé H-NAr₂ au squelette de base, avec dissociation du groupe partant.

12. Formulation contenant au moins un composé selon l'une ou plusieurs des revendications 1 à 10 et au moins un solvant et/ou au moins un autre composé organique ou inorganique.

13. Utilisation d'un composé selon l'une ou plusieurs des revendications 1 à 10 dans un dispositif électronique, en particulier dans un dispositif d'électroluminescence organique.

14. Dispositif électronique contenant au moins un composé selon l'une ou plusieurs des revendications 1 à 10.

15. Dispositif électronique selon la revendication 14, pour ce qui concerne lequel il s'agit d'un dispositif d'électroluminescence organique, **caractérisé en ce que** le composé selon l'une ou plusieurs des revendications 1 à 10 est utilisé en tant que matériau de matrice et/ou dans une couche de blocage de trous et/ou dans une couche de transport d'électrons.
